# EUROPEAN PATENT SPECIFICATION

(11) **EP 3 699 183 B1**
(45) Date of publication and mention of the grant of the patent: **24.07.2024**
(21) Application number: 18869066.3
(22) Date of filing: 10.08.2018
(51) Int. Cl.: C07F 7/10, A61K 8/898, A61Q 1/00, A61Q 1/02, A61Q 1/06, A61Q 1/08, A61Q 1/10, A61Q 1/12, A61Q 1/14, A61Q 5/00, A61Q 5/12, A61Q 15/00, A61Q 17/04, A61Q 19/00, A61Q 19/04, C08G 77/26, C08G 77/388, C08G 77/50

(54) **COSMETIC COMPRISING A UREA GROUP-CONTAINING ORGANOPOLYSILOXANE**
KOSMETIKUM ENTHALTEND EIN HARNSTOFFGRUPPENHALTIGES ORGANOPOLYSILOXAN
COSMETIQUE COMPRENANT UN ORGANOPOLYSILOXANE CONTENANT UN GROUPE URÉE

(30) Priority: 16.10.2017 JP 2017200490
(43) Date of publication of application: 26.08.2020
(73) Proprietor: Shin-Etsu Chemical Co., Ltd., Tokyo 100-0005 (JP)
(72) Inventor: KAMEI Masanao, Annaka-shi Gunma 379-0224 (JP); HIROKAMI Munenao, Annaka-shi Gunma 379-0224 (JP); ANDO Yuji, Annaka-shi Gunma 379-0224 (JP)
(74) Representative: Wibbelmann, Jobst
(86) International application number: PCT/JP2018/030108
(87) International publication number: WO 2019/077855

(56) References cited:
- EP-A1- 2 365 021
- WO-A1-2017/033080
- WO-A1-2017/033080
- CN-A- 108 218 906
- JP-A- 2005 029 575
- JP-A- 2006 510 750
- JP-A- 2006 510 750
- JP-A- 2011 026 493
- JP-A- H08 500 771
- JP-A- H08 500 771
- US-A- 4 344 763
- US-A1- 2017 277 002
- G. S. GOLDIN: "SINTEZ DISILILMOCHEVIN [SYNTHESIS OF DISILYL UREA]", ZHURNAL OBSHCHEI KHIMII, vol. 40, no. 4, 1970, pages 821 - 823, XP009519892, ISSN: 0044-460X

## Description

### TECHNICAL FIELD

The present invention relates to a novel cosmetic containing a urea group-containing organopolysiloxane and particularly to a cosmetic having excellent adhesion to the skin and hair, and good durability of each effect, while maintaining a feel of polysiloxane.

### BACKGROUND ART

Organopolysiloxanes have been used in various industrial fields such as electricity, electronics, automobile, office automation equipment, medical treatment, cosmetics, food, and construction. In cosmetics, they have also been widely used in skin care, makeup, hair care cosmetics, etc., because they have features such as a light feel, good spreadability, excellent water repellency, and high safety.

Organopolysiloxanes used for cosmetics is mainly a dimethylpolysiloxane, but by modifying various organic groups, they have been used as an oil, a surfactant, a thickener, a film forming agent, a surface treatment agent for powder, etc., while maintaining properties of siloxanes.

On the other hand, urea has been used as a lotion, a moisturizing cream, etc., and also been used as a keratin care component as another use, because it has a good affinity to the skin and a moisturizing function.

A block-type polymer in which polysiloxanes are linked via a urea bond (-NHCONH-) and an application of such a polymer to cosmetics have been known in large numbers. For example, a cosmetic in which a structured polymer via a urea bond (-NHCONH-) with organopolysiloxane groups is applied to keratin material (Patent Document 1), a thickener of a block-type polymer via a urea bond by reaction of a both-terminal amino modified silicone and hexamethylene diisocyanate (patent Document 2), etc., have been known.

In addition, heretofore, there has been a demand for a cosmetic having excellent cosmetic durability due to an improvement of an affinity to the skin and hair, while maintaining a light feel, good spreadability, water repellency, which are features of silicones.

### CITATION LIST

### PATENT LITERATURE

Patent Document 1: Japanese Patent Laid-Open No.2005-29575
Patent Document 2: Japanese Patent Laid-Open No.2011-225729

### SUMMARY OF INVENTION

### TECHNICAL PROBLEM

The present invention has been made in view of the above circumstances, and it is an object to provide a cosmetic comprising a urea group-containing organopolysiloxane having excellent cosmetic durability while maintaining a light feel and good spreadability, and a method for manufacturing the same, as well as a cosmetic containing the same.

### SOLUTION TO PROBLEM

In order to solve the problems, according to the present invention, a cosmetic comprising a urea group-containing organopolysiloxane represented by the following average composition formula (1) is provided.

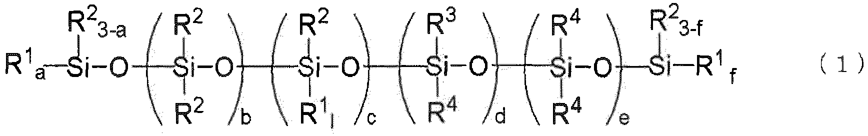

In the formula (1), R¹ is -R⁵-NHCONH₂ (R⁵ is a divalent hydrocarbon group having 2 to 10 carbon atoms), R²s are each independently any of a hydrogen atom, a hydroxy group, an alkoxy group having 1 to 3 carbon atoms, an alkyl group having 1 to 30 carbon atoms, a fluoroalkyl group having 1 to 30 carbon atoms, an aryl group having 6 to 30 carbon atoms, and an aralkyl group having 7 to 30 carbon atoms, R³ is R¹ or R², and R⁴ is an organopolysiloxane segment represented by the following average composition formula (2), 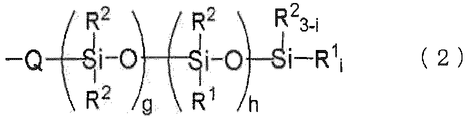
in the formula (2), R¹ and R² are as described above, and Q is an oxygen atom or a divalent hydrocarbon group having 1 to 3 carbon atoms,
in the average composition formula (1) and the general formula (2), "a", "f" and "i" are each independently 0 to 3, "b" is 0 to 5000, "c" is 0 to 500, "d" is 0 to 100, "e" is 0 to 100, "g" is 0 to 5000, and "h" is 0 to 500; provided that, the urea group-containing organopolysiloxane represented by the formula (1) has one or more R¹ in the molecule, when "c" is 0, "a" + "d" + "f" + "h" + "i" is 1 or more.

Such a urea group-containing organopolysiloxane, when used as a component of a cosmetic, forms a cosmetic having excellent cosmetic durability while maintaining a light feel and good spreadability.

In the present disclosure, a method for manufacturing the urea group-containing organopolysiloxane comprising: subjecting to addition reaction of an organohydrogenpolysiloxane and an unsaturated group-containing urea compound is also provided.

The urea group-containing organopolysiloxane used in the present invention can be manufactured by such addition reaction.

In this case, the unsaturated group-containing urea compound is preferably allyl urea.

It is preferable to use allyl urea because the urea group-containing organopolysiloxane of the present invention can be easily obtained.

In the present disclosure, a method for manufacturing the urea group-containing organopolysiloxane comprising: subjecting to reaction of an amino group-containing organopolysiloxane and urea is also provided.

The urea group-containing organopolysiloxane of the present invention can also be manufactured by such a reaction.

In the present invention, a cosmetic containing the urea group-containing organopolysiloxane is provided.

When a cosmetic contains such a urea group-containing organopolysiloxane, it will have excellent cosmetic durability due to an improvement of an affinity to the skin and hair, while maintaining a light feel, good spreadability, water repellency, which are features of silicones.

In this case, the cosmetic of the present invention can further contain water and can be in a form of an emulsion system of a water-in-oil emulsion or an oil-in-water emulsion.

The cosmetic of the present invention can take such an emulsion form depending on the purpose.

The cosmetic of the present invention can further contain powder.

Thus, the cosmetic of the present invention can contain powder depending on the purpose.

In addition, the cosmetic is preferably a skin care cosmetic, a makeup cosmetic, a hair cosmetic, an antiperspirant cosmetic, or an ultraviolet protection cosmetic.

The urea group-containing organopolysiloxane of the present invention can be suitably used as a material of such various cosmetics.

### ADVANTAGEOUS EFFECTS OF INVENTION

The urea group-containing organopolysiloxane used in the present invention can be synthesized by subjecting to addition reaction of a urea compound having an unsaturated group to an organohydrogenpolysiloxane, and when the resulting urea group-containing organopolysiloxane is used for a cosmetic, it will have excellent cosmetic durability due to an improvement of an affinity to the skin and hair, while maintaining a light feel, good spreadability, water repellency, which are features of silicones.

### DESCRIPTION OF EMBODIMENTS

As described above, a polymer via a urea bond (-NHCONH-) is known, but an organopolysiloxane having a urea group (-NHCONH₂) in the molecule is not known.

As a result of intensive studies to accomplish the objects mentioned above, the present inventor has found that use of the organopolysiloxane having a urea group in the molecule represented by the following average composition formula (1) as a component of a cosmetic results in a cosmetic having excellent cosmetic durability due to an improvement of an affinity to the skin and hair, while maintaining a light feel, good spreadability, water repellency, which are features of silicones, and has accomplished the present invention. Hereinafter, the urea group-containing organopolysiloxane of the present invention will be described in detail.

That is, the present invention uses a urea group-containing organopolysiloxane represented by the following average composition formula (1).

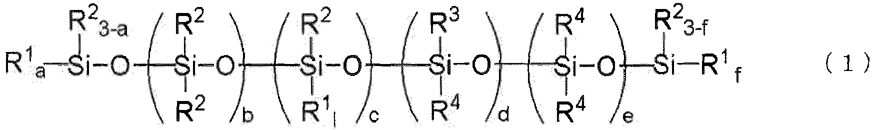

In the formula (1), R¹ is -R⁵-NHCONH₂ (R⁵ is a divalent hydrocarbon group having 2 to 10 carbon atoms), R²s are each independently any of a hydrogen atom, a hydroxy group, an alkoxy group having 1 to 3 carbon atoms, an alkyl group having 1 to 30 carbon atoms, a fluoroalkyl group having 1 to 30 carbon atoms, an aryl group having 6 to 30 carbon atoms, and an aralkyl group having 7 to 30 carbon atoms, R³ is R¹ or R², and R⁴ is an organopolysiloxane segment represented by the following average composition formula (2), 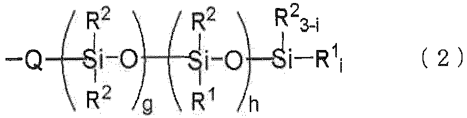
in the formula (2), R¹ and R² are as described above, and Q is an oxygen atom or a divalent hydrocarbon group having 1 to 3 carbon atoms,
in the average composition formula (1) and the general formula (2), "a", "f" and "i" are each independently 0 to 3, "b" is 0 to 5000, "c" is 0 to 500, "d" is 0 to 100, "e" is 0 to 100, "g" is 0 to 5000, and "h" is 0 to 500; provided that, the urea group-containing organopolysiloxane represented by the formula (1) has one or more R¹ in the molecule, when "c" is 0, "a" + "d" + "f" + "h" + "i" is 1 or more.

In the general formula (1), R¹ is the general formula -R⁵-NHCONH₂, and R⁵ is a divalent hydrocarbon group having 2 to 10 carbon atoms. R⁵ is specifically an ethylene group, a propylene group, a hexamethylene group, a decamethylene group, etc., and preferably an ethylene group and a propylene group, and more preferably a propylene group.

R²s are each independently any of a hydrogen atom, a hydroxy group, an alkoxy group having 1 to 3 carbon atoms, an alkyl group having 1 to 30 carbon atoms, a fluoroalkyl group having 1 to 30 carbon atoms, an aryl group having 6 to 30 carbon atoms, and an aralkyl group having 7 to 30 carbon atoms, and specifically include a methyl group, an ethyl group, a propyl group, a hexyl group, a heptyl group, an octyl group, a nonyl group, a decyl group, a dodecyl group, and a hexadecyl group, and are preferably a methyl group, a hexyl group, a dodecyl group, and a hexadecyl group, and more preferably a methyl group.

R³ is R¹ or R², R⁴ is an organopolysiloxane segment represented by the average composition formula (2).

In the formula (2), R¹, R² are the same as described above, and Q is an oxygen atom or a divalent hydrocarbon group having 1 to 3 carbon atoms, and specifically a methylene group, an ethylene group and a propylene group, and preferably an oxygen atom or an ethylene group.

In the average composition formula (1) and the general formula (2), "a", "f" and "i" are each independently 0 to 3, and preferably 0 or 1, "b" is 0 to 5000, and preferably 0 to 100, "c" is 0 to 500, and preferably 0 to 50, "d" is 0 to 100, and preferably 0 to 20, "e" is 0 to 100, and preferably 0 to 20, "g" is 0 to 5000, and preferably 1 to 100, and "h" is 0 to 500, and preferably 0 to 20. Provided that, these values are a value satisfying the condition that the urea group-containing organopolysiloxane represented by the formula (1) has one or more R¹ in the molecule, when "c" is 0, "a" + "d" + "f" + "h" + "i" is 1 or more.

The urea group-containing organopolysiloxane used in the present invention can be manufactured by subjecting to addition reaction of an organohydrogenpolysiloxane and an unsaturated group-containing urea compound. Examples of the unsaturated group-containing urea compound include specifically vinyl urea, allyl urea, propenyl urea, and hexenyl urea, and preferably vinyl urea and allyl urea, and more preferably allyl urea.

The organohydrogenpolysiloxane used herein may be either linear, cyclic, or blanched. In addition, a binding site of the Si-H groups is not particularly limited, and may be either a side-chain or a terminal.

In addition, it is desirable to carry out the above addition reaction in the presence of a platinum-based catalyst or a rhodium-based catalyst, and specifically a chloroplatinic acid, an alcohol-modified chloroplatinic acid, a chloroplatinic acid-vinyl siloxane complex, etc. are suitably used. Incidentally, an amount of the catalyst used can be a catalyst amount, and particularly an amount of platinum or rhodium is 200 ppm or less, and preferably 20 ppm or less.

The above addition reaction may be carried out in an organic solvent depending on necessity, examples of the organic solvent include a lower alcohol such as methanol, ethanol, and isopropyl alcohol; an aromatic hydrocarbon such as toluene and xylene; an aliphatic or an alicyclic hydrocarbon such as n-pentane, n-hexane, and cyclohexane; a halogenated hydrocarbon such as dichloromethane, chloroform, and carbon tetrachloride; an ether compound such as tetrahydrofuran and dioxane; and ketones such as acetone and methyl ethyl ketone, but preferably a lower alcohol compound and an ether compound.

The addition reaction conditions are not particularly limited, but they are suitable to react at 30°C to 150°C for 1 to 20 hours.

In addition, the urea group-containing organopolysiloxane of the present invention can also be manufactured by subjecting to reaction of an amino group-containing organopolysiloxane and urea. The amino group-containing organopolysiloxane used herein may be either linear, chained, or blanched, a binding site of the amino group is not particularly limited, and may be either a side-chain or a terminal.

In addition, the above reaction may be carried out in an organic solvent depending on necessity, examples of the organic solvent include a lower alcohol such as methanol, ethanol, and isopropyl alcohol; an aromatic hydrocarbon such as toluene and xylene; an aliphatic or an alicyclic hydrocarbon such as n-pentane, n-hexane, and cyclohexane; and an ether compound such as tetrahydrofuran and dioxane.

The reaction conditions are not particularly limited, but they are suitable to react at 80°C to 150°C for 1 to 40 hours, and more preferably to react at 100 to 130°C.

The urea group-containing organopolysiloxane used in the present invention can be used in various applications, but is particularly suitable as a raw material for all cosmetics externally used for the skin or hair. In this case, a formulation amount of the urea group-containing organopolysiloxane of the average composition formula (1) is suitable in the range of 0.1 to 70 parts by mass based on the entire cosmetic.

In the cosmetic of the present invention, one or more oils can be formulated depending on the purpose. Any solid, semi-solid, or liquid oil can be used as long as it is used in the usual cosmetics.

Examples of natural animal and vegetable fats and oils and semisynthetic fats and oils include avocado oil, linseed oil, almond oil, insect wax, perilla oil, olive oil, cacao butter, kapok wax, kaya oil, carnauba wax, liver oil, candelilla wax, purified candelilla wax, beef tallow, beef foot fat, beef bone fat, hardened beef tallow, apricot kernel oil, whale wax, hardened oil, wheat germ oil, sesame oil, rice germ oil, rice bran oil, sugarcane wax, Camellia sasanqua oil, sunflower oil, shea butter, Chinese tung oil, cinnamon oil, jojoba wax, squalane, squalene, shellac wax, turtle oil, soybean oil, tea seed oil, camellia oil, evening primrose oil, corn oil, lard, rapeseed oil, Japanese tung oil, bran wax, germ oil, horse fat, persic oil, palm oil, palm kernel oil, castor oil, hardened castor oil, castor oil fatty acid methyl ester, sunflower oil, grape oil, bayberry wax, jojoba oil, macadamia nut oil, bees wax, mink oil, meadowfoam oil, cottonseed oil, cotton wax, Japan wax, Japan wax kernel oil, montan wax, coconut oil, hardened coconut oil, tri-coconut oil fatty acid glyceride, mutton tallow, peanut oil, lanolin, liquid lanolin, reduced lanolin, lanolin alcohol, hard lanolin, lanolin acetate, lanolin acetate alcohol, lanolin fatty acid isopropyl, POE lanolin alcohol ether, POE lanolin alcohol acetate, lanolin fatty acid polyethylene glycol, POE hydrogenated lanolin alcohol ether, and egg yolk oil. Provided that, POE means polyoxyethylene.

Examples of hydrocarbon oils include linear, branched, and further volatile hydrocarbon oils, and specifically ozokerite, an α-olefin oligomer, light isoparaffin, isododecane, isohexadecane, light liquid isoparaffin, squalane, synthetic squalane, vegetable squalane, squalene, ceresin, paraffin, paraffin wax, polyethylene wax, polyethylene-polypropylene wax, an (ethylene/propylene/styrene) copolymer, a (butylene/propylene/styrene) copolymer, liquid paraffin, liquid isoparaffin, pristane, polyisobutylene, hydrogenated polyisobutene, microcrystalline wax, and Vaseline; and examples of higher fatty acids include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.

Examples of higher alcohols include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecynol, cholesterol, phytosterol, POE cholesterol ether, monostearyl glycerol ether (batyl alcohol), and monooleyl glyceryl ether (selachyl alcohol).

Examples of ester oils include diisobutyl adipate, 2-hexyldecyl adipate, di-2-heptylundecyl adipate, N-alkylglycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dioctanoate, neopentyl glycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isononyl isononanate, isotridecyl isononanate, isopropyl palmitate, 2-ethylhexyl palmitate, 2-hexyldecyl palmitate, 2-heptylundecyl palmitate, cholesteryl 12-hydroxystearate, dipenta-erythritol fatty acid ester, isopropyl myristate, octyldodecyl myristate, 2-hexyldecyl myristate, myristyl myristate, hexyldecyl dimethyloctanoate, ethyl laurate, hexyl laurate, N-lauroyl-L-glutamic acid-2-octyldodecyl ester, lauroyl sarcosine isopropyl ester, diisostearyl malate; and examples of glyceride oils include acetoglyceryl, glyceryl triisooctanoate, glyceryl triisostearate, glyceryl triisopalmitate, glyceryl tribehenate, glyceryl monostearate, glyceryl di-2-heptylundecanoate, glyceryl trimyristate, and diglyceryl myristate/isostearate.

Examples of silicone oils include a linear or branched organopolysiloxane of low viscosity to high viscosity such as dimethylpolysiloxane, tristrimethylsiloxymethylsilane, caprylyl methicone, phenyl trimethicone, tetrakistrimethylsiloxysilane, methylphenylpolysiloxane, methylhexylpolysiloxane, methylhydrogenpolysiloxane, and a dimethylsiloxane-methylphenylsiloxane copolymer; a cyclic organopolysiloxane such as octamethylcyclotetrasiloxane, decamethylcyclopentasiloxane, dodecamethylcyclohexasiloxane, tetramethyltetrahydrogencyclotetrasiloxane, and tetramethyltetraphenylcyclotetrasiloxane; an amino-modified organopolysiloxane, a pyrrolidone-modified organopolysiloxane, a pyrrolidone-carboxylic acid-modified organopolysiloxane; a silicone rubber such as a high polymerization degree gum-like dimethylpolysiloxane, a gum-like amino-modified organopolysiloxane, and a gum-like dimethylsiloxane·methylphenylsiloxane copolymer; and a cyclic organopolysiloxane solution of silicone gum or rubber, trimethylsiloxysilicic acid, a cyclic siloxane solution of trimethylsiloxysilicic acid, a higher alkoxy-modified silicone such as stearoxy silicone, a higher fatty acid-modified silicone, an alkyl-modified silicone, a long-chain alkyl-modified silicone, an amino acid-modified silicone, a fluorine-modified silicone, a silicone resin, and a dissolved product of a silicone resin. Examples of fluorine-based oils include perfluoropolyether, perfluorodecalin, and perfluorooctane.

The silicone oil can be formulated as a mixed system with the oil other than the silicone oil. In the present invention, even if the oil is a mixed system oil of the silicone oil and the oil other than the silicone oil, a cosmetic that does not impair the feel of the silicone can be provided by using the urea group-containing organopolysiloxane of the average composition formula (1).

A formulation amount of these oils depends on the agent system, but it is suitable in the range of 1 to 98% by mass based on the entire cosmetic.

In the cosmetic of the present invention, an ultraviolet absorber can be formulated depending on the purpose. A formulation amount thereof depends on the agent system, but it is suitable in the range of 1 to 30% by mass based on the entire cosmetic.

Examples of the ultraviolet absorber include a benzoic acid-based ultraviolet absorber such as para-aminobenzoic acid, ethyl para-amino benzoate, glyceryl para-amino benzoate, and octyl para-dimethyl benzoate; an anthranilic acid-based ultraviolet absorber such as methyl anthranilate; a salicylic acid-based ultraviolet absorber such as methyl salicylate, octyl salicylate, trimethylcyclohexyl salicylate, ethylene glycol salicylate, and phenyl salicylate; a cinnamic acid-based ultraviolet absorber such as benzyl cinnamate, octyl para-methoxycinnamate, and 2-ethoxyethyl para-methoxycinnamate; a benzophenone-based ultraviolet absorber such as 2,4-dihydroxybenzophenone, tetrahydroxybenzophenone, and hydroxymethoxybenzophenone; an urocanic acid-based ultraviolet absorber such as urocanic acid, and ethyl urocanate; a dibenzoylmethane-based ultraviolet absorber such as 4-isopropyl dibenzoylmethane, and 4-t-butyl-4'-methoxy-dibenzoylmethane, phenylbenzimidazole sulfonic acid, and a triazine derivative.

The ultraviolet absorber can be formulated as a mixed system with the oil. In the present invention, even if the formulation is a mixed system of such an ultraviolet absorber and the oil, a cosmetic that does not impair the feel of the silicone can be provided by using the urea group-containing organopolysiloxane of the average composition formula (1).

In the cosmetic of the present invention, water can be formulated depending on the purpose. A formulation amount thereof depends on the agent system, but it is suitable in the range of 1 to 95% by mass based on the entire cosmetic. Thus, when the cosmetic contains water, it is in a form of an emulsion system of a water-in-oil emulsion or an oil-in-water emulsion.

In the cosmetic of the present invention, one or more compounds having an alcoholic hydroxyl group in the molecular structure can also be used depending on the purpose.

Examples of the compound having an alcoholic hydroxyl group that can be added in the present invention include a lower alcohol such as ethanol and isopropanol; a sugar alcohol such as sorbitol and maltose; a sterol such as cholesterol, sitosterol, phytosterol and lanosterol; and a polyhydric alcohol such as butylene glycol, propylene glycol, dibutylene glycol, and pentylene glycol. As a formulation amount thereof, it is suitable in the range of 0.1 to 98% by mass based on the entire cosmetic.

In the cosmetic of the present invention, one or more water-soluble or water-swellable polymers can also be used depending on the purpose. For example, there are a plant-based polymer such as gum arabic, tragacanth, galactan, carob gum, guar gum, karaya gum, carrageenan, pectin, agar, quince seed (marmelo), starch (rice, corn, potato, wheat, etc.), algae colloid, trant gum, and locust bean gum; a microorganism-based polymer such as xanthan gum, dextran, succinoglucan, and pullulan; an animal-based polymer such as collagen, casein, albumin, and gelatin; a starch-based polymer such as carboxymethyl starch, and methylhydroxypropyl starch; a cellulose-based polymer such as methyl cellulose, ethyl cellulose, methylhydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose and cellulose powder; an alginic acid-based polymer such as sodium alginate and alginic acid propylene glycol ester; a vinyl-based polymer such as polyvinyl methyl ether and a carboxy vinyl polymer; a polyoxyethylene-based polymer, a polyoxyethylene polyoxypropylene copolymer-based polymer; an acrylic-based polymer such as sodium polyacrylate, polyethyl acrylate, polyacrylamide, and an acryloyldimethyltaurine salt copolymer; another synthetic water-soluble polymer such as polyethyleneimine and a cationic polymer; an inorganic-based water-soluble polymer such as bentonite, magnesium aluminum silicate, montmorillonite, beidellite, nontronite, saponite, hectorite, and silicic anhydride, etc. In addition, these water-soluble polymers include a film-forming agent such as polyvinyl alcohol and polyvinyl pyrrolidone. As a formulation amount thereof, it is suitable in the range of 0.1 to 25% by mass based on the entire cosmetic.

In the cosmetic of the present invention, one or more powders and/or colorants can also be used depending on the purpose. As the powder and/or the colorant, any of the materials can also be used as long as it is used in the usual cosmetics, regardless of its shape (spherical, needle, plate, etc.), particle diameter (fumed, fine particles, pigment grade, etc.), or particulate structure (porous, nonporous, etc.), and examples of the powder and/or the colorant include an inorganic powder, an organic powder, a surfactant metal salt powder, a colored pigment, a pearl pigment, a metal powder pigment, a tar pigment, a natural pigment.

Specifically, the inorganic powder is a powder selected from titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lithia mica, silicic acid, silicic anhydride, aluminum silicate, magnesium silicate, magnesium aluminum silicate, calcium silicate, barium silicate, strontium silicate, metal tungstate, hydroxyapatite, vermiculite, Higilite, bentonite, montmorillonite, hectorite, zeolite, ceramic powder, dibasic calcium phosphate, alumina, aluminum hydroxide, boron nitride, boron nitride, silica, etc.; and the organic powder is a powder selected from polyamide powder, polyester powder, polyethylene powder, polypropylene powder, polystyrene powder, polyurethane, benzoguanamine powder, polymethylbenzoguanamine powder, tetrafluoroethylene powder, polymethyl methacrylate powder, cellulose, silk powder, nylon powder, 12 nylon, 6 nylon, silicone powder, styrene·acrylic acid copolymer, divinylbenzene·styrene copolymer, vinyl resin, urea resin, phenol resin, fluorine resin, silicon resin, acrylic resin, melamine resin, epoxy resin, polycarbonate resin, microcrystalline fiber powder, starch powder, lauroyl lysine, etc.; and the surfactant metal salt powder (metallic soap) is a powder selected from zinc stearate, aluminum stearate, calcium stearate, magnesium stearate, zinc myristate, magnesium myristate, zinc cetyl phosphate, calcium cetyl phosphate, zinc sodium cetyl phosphate, etc.; and the colored pigment is a powder selected from an inorganic red pigment such as iron oxide, iron hydroxide and iron titanate; an inorganic brown pigment such as γ-iron oxide; an inorganic yellow pigment such as yellow iron oxide and loess; an inorganic black pigment such as black iron oxide and carbon black; an inorganic violet pigment such as manganese violet and cobalt violet; an inorganic green pigment such as chromium hydroxide, chromium oxide, cobalt oxide, and cobalt titanate; an inorganic blue pigment such as prussian blue and ultramarine blue; that obtained by laking a tar pigment, that obtained by laking a natural pigment, a synthetic resin powder obtained by combining these powders, etc.; and the pearl pigment is a powder selected from titanium oxide-coated mica, titanium oxide-coated mica, bismuth oxychloride, titanium oxide-coated bismuth oxychloride, titanium oxide-coated talc, fish scale foil, titanium oxide-coated coloring mica, etc.; and the metal powder pigment is a powder selected from aluminum powder, copper powder, stainless powder, etc.; and the tar pigment is a powder selected from Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, Orange No. 207, etc.; and the natural pigment is a powder selected from carminic acid, laccaic acid, carthamin, brazilin, crocin, etc.

Specific examples of each are as described above, as these powders, those in which powders are combined, or those treated with a general oil, a silicone oil, a fluorine compound, a surfactant, etc., can also be used, and one or more of those treated with a hydrolyzable silyl group or an alkyl group having a hydrogen atom directly bonded to a silicon atom, a linear and/or branched organopolysiloxane having a hydrolyzable silyl group or a hydrogen atom directly bonded to a silicon atom, a linear and/or branched organopolysiloxane having a hydrolyzable silyl group or a hydrogen atom directly bonded to a silicon atom and being co-modified by a long chain alkyl, a linear and/or branched organopolysiloxane having a hydrolyzable silyl group or a hydrogen atom directly bonded to a silicon atom and being co-modified by polyoxyalkylene, and an acrylic-silicone-based copolymer having a hydrolyzable silyl group or a hydrogen atom directly bonded to a silicon atom, etc., can also be used depending on necessity.

In addition, as a formulation amount thereof, it is suitable in the range of 0.1 to 99% by mass based on the entire cosmetic. Particularly, as a formulation amount in the case of a powdered solid cosmetic, it is suitable in the range of 80 to 99% by mass based on the entire cosmetic.

In the cosmetic of the present invention, one or more surfactants can also be used depending on the purpose. As such a surfactant, there are anionic, cationic, nonionic and amphoteric active agents, but it is not particularly limited, and any material can be used as long as it is used in the usual cosmetics.

In the following, specific examples of the anion surfactant include a fatty acid soap such as sodium stearate and triethanolamine palmitate, an alkyl ether carboxylic acid and a salt thereof, a salt of a condensate of an amino acid and a fatty acid, an alkane sulfonic acid salt, an alkene sulfonic acid salt, a fatty acid ester sulfonic acid salt, a fatty acid amide sulfonic acid salt, a formalin condensation-based sulfonic acid salt, a sulfuric acid ester salt such as an alkyl sulfuric acid ester salt, a secondary higher alcohol sulfuric acid ester salt, an alkyl and allyl ether sulfuric acid ester salt, a sulfuric acid ester salt of a fatty acid ester, a sulfuric acid ester salt of a fatty acid alkylol amide, sulfuric acid ester salts such as turkey red oil, an alkyl phosphoric acid salt, an ether phosphoric acid salt, an alkyl aryl ether phosphoric acid salt, an amide phosphoric acid salt, an N-acyl lactic acid salt, an N-acyl sarcosine salt, and an N-acylamino acid-based active agent; and examples of the cationic surfactant include an amine salt such as an alkylamine salt, a polyamine and an amino alcohol fatty acid derivative, an alkyl quaternary ammonium salt, an aromatic quaternary ammonium salt, a pyridium salt, and an imidazolium salt.

Examples of the nonionic surfactant include a sorbitan fatty acid ester, a glycerol fatty acid ester, a polyglycerol fatty acid ester, a propylene glycol fatty acid ester, a polyethylene glycol fatty acid ester, a sucrose fatty acid ester, a methyl glucoside fatty acid ester, an alkyl polyglucoside, a polyoxyethylene alkyl ether, a polyoxypropylene alkyl ether, a polyoxyethylene alkyl phenyl ether, a polyoxyethylene fatty acid ester, a polyoxyethylene sorbitan fatty acid ester, a polyoxyethylene sorbitol fatty acid ester, a polyoxyethylene glycerol fatty acid ester, a polyoxyethylene propylene glycol fatty acid ester, a polyoxyethylene castor oil, a polyoxyethylene hardened castor oil, a polyoxyethylene phytostanol ether, a polyoxyethylene phytosterol ether, a polyoxyethylene cholestanol ether, a polyoxyethylene cholesteryl ether, a linear or branched polyoxyalkylene-modified organopolysiloxane, a linear or branched polyoxyalkylene·alkyl-co-modified organopolysiloxane, a linear or branched polyglycerol-modified organopolysiloxane, a linear or branched polyglycerol·alkyl-co-modified organopolysiloxane, an alkanol amide, a sugar ether, and a sugar amide; and examples of the amphoteric surfactant include betaine, phosphatidylcholine, an aminocarboxylic acid salt, an imidazoline derivative, and an amideamine type.

Among these surfactants, a linear or branched polyoxyalkylene-modified organopolysiloxane, a linear or branched polyoxyalkylene·alkyl-co-modified organopolysiloxane, a linear or branched polyglycerol-modified organopolysiloxane, a linear or branched polyglycerol·alkyl-co-modified organopolysiloxane are preferred, and more specifically the silicone surfactant represented by the following average composition formula (3) are preferred.

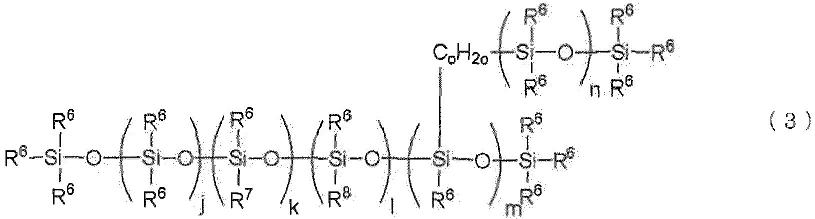

In the formula (3), R⁶s are each independently a substituted or unsubstituted monovalent hydrocarbon group having 1 to 30 carbon atoms, R⁷s are each independently the group represented by the following (4) or the following (5); provided that, the following (4) includes both a block type and a random type, the polyglycerol of the following formula (5) includes isomers,

-CₚH₂ₚO (C₂H₄O)_{q} (C₃H₆O)ᵣ R⁹ (4)

-CₚH₂ₚO (C₃H₅(OR¹⁰)O)ₛ R¹¹ (5)

R⁸ is a monovalent hydrocarbon group having 6 to 30 carbon atoms, R⁹, R¹⁰, and R¹¹ are groups selected from a hydrogen atom or a monovalent hydrocarbon group having 1 to 6 carbon atoms, and at least one of R¹⁰ or R¹¹ is always a hydrogen atom. "j" is an integer of 0 to 200, "k" is an integer of 1 to 30, "l" is an integer of 0 to 50, "m" is an integer of 0 to 30, "n" is an integer of 1 to 100, "o" is an integer of 2 to 10, "p" is an integer of 2 to 10, "q" is an integer of 0 to 50, "r" is an integer of 0 to 50; provided that, q + r ≥ 1. "s" is an integer of 1 to 6.

A compound having the group represented by the formula (4) is a silicone surfactant using a polyether as a hydrophilic moiety. A compound having the group represented by the formula (5) is a silicone surfactant using a polyglycerol as a hydrophilic moiety.

In the formula (3), R⁶s are each independently a substituted or unsubstituted monovalent hydrocarbon group having 1 to 30 carbon atoms, preferably an alkyl group having 1 to 30 carbon atoms, a halogen-substituted alkyl group having 1 to 15 carbon atoms, an aryl group having 6 to 12 carbon atoms. Examples of the R⁶ include an alkyl group such as a methyl group, an ethyl group, a propyl group, a butyl group, a pentyl group, a hexyl group, a heptyl group, an octyl group, a nonyl group, a decyl group, and a stearyl group; a cycloalkyl group such as a cyclopentyl group and a cyclohexyl group; an aryl group such as a phenyl group, an ethylphenyl group and a propylphenyl group; and a hydrocarbon group in which part or all of hydrogen atoms bonded to carbon atoms of these groups are substituted with atoms such as halogen atoms (fluorine atom, chlorine atom, bromine atom, iodine atom). Examples of the group substituted with a fluorine atom include a fluorine-substituted alkyl group such as a trifluoropropyl group and a hepta deca fluoro decyl group. Preferably, it is an alkyl group having 1 to 15 carbon atoms and a phenyl group.

In the formula (4) and (5), R⁹, R¹⁰, and R¹¹ are groups selected from a hydrogen atom or a hydrocarbon group having 1 to 6 carbon atoms. Examples of the hydrocarbon group include a methyl group, an ethyl group, a propyl group, a butyl group, a pentyl group, and a hexyl group, preferably they are a hydrogen atom or a methyl group, a butyl group. In addition, at least one of R¹⁰ or R¹¹ is always hydrogen.

"j" is 0 to 200, and preferably 0 to 100, "k" is 1 to 30, and preferably 1 to 20, "l" is 0 to 50, and preferably 0 to 20, "m" is 0 to 30, and preferably 0 to 10, "n" is 1 to 100, and preferably 1 to 50, "o" is 2 to 10, and preferably 2 to 4, "p" is 2 to 10, and preferably 2 to 4, "q" is 0 to 50, and preferably 0 to 30, "r" is 0 to 50, and preferably 0 to 30; provided that, q + r ≥ 1, "s" is 1 to 6, and preferably 1 to 4.

Examples of the compound having the group represented by the formula (4) include KF-6011, KF-6011P, KF-6012, KF-6013, KF-6015, KF-6017, KF-6017P, KF-6028, KF-6028P, KF-6038, and KF-6043 (manufactured by Shin-Etsu Chemical Co., Ltd.). Examples of the compound having the group represented by the formula (5) include KF-6100, KF-6104, and KF-6105 (manufactured by Shin-Etsu Chemical Co., Ltd.).

In addition, in these surfactants, a content of a hydrophilic polyoxyalkylene group or a polyglycerol residue preferably occupies 10 to 70% by mass in the molecule, and a formulation amount thereof is in the range of 0.1 to 20% by mass, particularly suitable in the range of 0.2 to 10% by mass based on the entire cosmetic.

In the cosmetic of the present invention, one or more silicone resins can also be used depending on the purpose.

The silicone resin is preferably an acrylic silicone resin of an acrylic / silicone graft or block copolymer. In addition, an acrylic silicone resin containing at least one selected from anionic moieties such as a pyrrolidone moiety, a long chain alkyl moiety, a polyoxyalkylene moiety, a fluoroalkyl moiety, and a carboxylic acid in the molecule can also be used.

Examples of the acrylic-silicone graft copolymer include KP-541, KP-543, KP-545, KP-549, KP-550, KP-545L, KP-561P, KP-562P, KP-575, and P-578 (manufactured by Shin-Etsu Chemical Co., Ltd.).

Further, the silicone resin is preferably a silicone network compound consisting of a resin composed of a R₃SiO_{0.5} unit and a SiOz unit, a resin composed of a R₃SiO_{0.5} unit, a R₂SiO unit, and a SiO₂ unit, a resin composed of a R₃SiO_{0.5} unit and a RSiO_{1.5} unit, a resin composed of a R₃SiO_{0.5} unit, a R₂SiO unit, and a RSiO_{1.5} unit, and a resin composed of a R₃SiO_{0.5} unit, a R₂SiO unit, a RSiO_{1.5} unit, and a SiO₂ unit (R is a univalent hydrocarbon group). In addition, the silicone network compound containing at least one selected from a pyrrolidone moiety, a long chain alkyl moiety, a polyoxyalkylene moiety, a fluoroalkyl moiety, and an amino moiety in the molecule can also be used.

I Examples of the silicone resin include KF-7312J, KF-7312K, KF-7312T, X-21-5249, X-21-5250, KF-9021, X-21-5595, and X-21-5616 (manufactured by Shin-Etsu Chemical Co., Ltd.).

As a formulation amount in the case of using a silicone resin such as an acrylic silicone resin and a silicone network compound, 0.1 to 20% by mass based on the total amount of the cosmetic is preferred, 1 to 10% by mass is further preferred.

In the cosmetic of the present invention, a composition consisting of one or more crosslinked organopolysiloxanes and an oil that is liquid at room temperature can also be used depending on the purpose. The crosslinked organopolysiloxane preferably swells with respect to the liquid oil by containing the liquid oil in an amount of its own weight or more. As the liquid oil, the above liquid silicone oil, a hydrocarbon oil, an ester oil, a natural animal and vegetable oil, a semisynthetic oil, etc., and a fluorine-based oil can be used, and examples of the liquid oil include a low viscosity silicone oil with 0.65 mm²/sec (25°C) to 100.0 mm²/sec (25°C); a hydrocarbon oil such as liquid paraffin, squalane, isododecane, and isohexadecane; a glyceride oil such as trioctanoin; an ester oil such as isotridecyl isononanoate, N-acyl glutamic acid ester, and lauroyl sarcosinic acid ester; and a natural animal and vegetable oil such as macadamia nut oil. In addition, a crosslinking agent for obtaining the crosslinked organopolysiloxane preferably has two or more vinylic reactive sites in the molecule, and forms a crosslinked structure by reacting with a hydrogen atom directly bonded to a silicon atom. Examples of the crosslinking agent having two or more vinylic reactive sites in the molecule include an organopolysiloxane having two or more vinyl groups in the molecule, a polyoxyalkylene having two or more allyl groups in the molecule, a polyglycerol having two or more allyl groups in the molecule, and α, ω-alkenyldiene.

In addition, a crosslinked organopolysiloxane containing at least one selected from the group consisting of a polyoxyalkylene moiety, a polyglycerol moiety, a long chain alkyl moiety, an alkenyl moiety, an aryl moiety, and a fluoroalkyl moiety in the crosslinking molecule can also be used.

Examples of the composition consisting of the crosslinked organopolysiloxanes and the oil that is liquid at room temperature include KSG-15, KSG-1510, KSG-16, KSG-1610, KSG-18A, KSG-41A, KSG-42A, KSG-43, KSG-44, KSG-045Z, KSG-210, KSG-310, KSG-320, KSG-330, KSG-340, KSG-320Z, KSG-350Z, KSG-360Z, KSG-710, KSG-810, KSG-820, KSG-840, KSG-840, KSG-820Z, KSG-850Z (manufactured by Shin-Etsu Chemical Co., Ltd.).

As a formulation amount in the case of using the composition consisting of the crosslinked organopolysiloxanes and the oil that is liquid at room temperature, 0.1 to 80% by mass based on the total amount of the cosmetic is preferred, 1 to 50% by mass is further preferred.

In the cosmetic of the present invention, one or more silicone waxes can also be used depending on the purpose. The silicone wax is a silicone-modified olefin wax obtained by subjecting to addition reaction of an olefin wax having an unsaturated group consisting of an α-olefin and a diene, and an organohydrogenpolysiloxane having one or more SiH bonds in one molecule. As the α-olefin of the olefin wax, carbon number of 2 to 12 such as ethylene, propylene, 1-butene, 1-hexene, and 4-methyl 1-pentene is preferred, as the diene, butadiene, isoprene, 1.4 hexadiene, vinyl norbornene, ethylidene norbornene, and dicyclopentadiene, etc., are preferred. As the organohydrogenpolysiloxane having a SiH bond, that having a linear or siloxane branched structure can be used.

Further, to the cosmetic of the present invention, a component to be used for usual cosmetics, an oil-soluble gelling agent, an organically modified clay mineral, a resin, an antiperspirant, an ultraviolet absorptive scattering agent, a moisturizing agent, an antiseptic, an antimicrobial agent, a perfume, salts, an antioxidant, a pH adjuster, a chelating agent, a refrigerant, an anti-inflammatory agent, a skin beautifying component (a whitening agent, a cell activator, a rough skin ameliorating agent, a blood circulation promoter, a skin astringent, an antiseborrheic drug, etc.), vitamins, amino acids, a nucleic acid, a hormone, an inclusion compound, a solidifying agent for hair, etc., can be added.

Examples of the oil-soluble gelling agent include a gelling agent selected from a metallic soap such as aluminum stearate, magnesium stearate, and zinc myristate; an amino acid derivative such as N-lauroyl-L-glutamic acid and α,γ-di-n-butylamine; a dextrin fatty acid ester such as dextrin palmitic acid ester, dextrin stearic acid ester, and dextrin 2-ethylhexanoic acid / palmitic acid ester; a sucrose fatty acid ester such as sucrose palmitic acid ester and sucrose stearic acid ester; a fructo-oligosaccharide fatty acid ester such as fructo-oligosaccharide stearic acid ester and fructo-oligosaccharide 2-ethylhexanoic acid ester; a benzylidene derivative of sorbitol such as monobenzylidene sorbitol and dibenzylidene sorbitol; and an organically modified clay mineral such as dimethylbenzyldodecylammonium montmorillonite clay and dimethyldioctadecylammonium montmorillonite clay, etc.

Examples of the antiperspirant include an antiperspirant selected from aluminum chlorohydrate, aluminum chloride, aluminum sesquichlorohydrate, zirconyl hydroxychloride, aluminum zirconium hydroxychloride, aluminum zirconium glycine complex, etc.

Examples of the ultraviolet absorptive scattering agent include a powder which absorbs or scatters ultraviolet rays such as fine particulate titanium oxide, fine particulate iron-containing titanium oxide, fine particulate zinc oxide, fine particulate cerium oxide, and a composite thereof.

Examples of the moisturizing agent include glycerol, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate, polyoxyethylene methyl glucoside, polyoxypropylene methyl glucoside, egg yolk lecithin, soybean lecithin, phosphatidyl choline, phosphatidyl ethanolamine, phosphadithyl serine, phosphatidyl glycerol, phosphatidylinositol, and sphingo-phospholipid.

Examples of the antimicrobial preservative include paraoxybenzoic acid alkyl ester, benzoic acid, sodium benzoate, sorbic acid, potassium sorbate, and phenoxyethanol; and examples of the antimicrobial agent include benzoic acid, salicylic acid, carbolic acid, sorbic acid, paraoxybenzoic acid alkyl ester, parachlorometacresol, hexachlorophene, benzalkonium chloride, chlorhexidine chloride, trichloro¬carbanilide, a photosensitizer, and phenoxyethanol.

Examples of the salts include an inorganic salt, an organic salt, an amine salt and an amino acid salt. Examples of the inorganic salt include a sodium salt, a potassium salt, a magnesium salt, a calcium salt, an aluminum salt, a zirconium salt, a zinc salt of an inorganic acid such as hydrochloric acid, sulfuric acid, carbonic acid, and nitric acid; and examples of the organic salt include a salt of an organic acid such as acetic acid, dehydroacetic acid, citric acid, malic acid, succinic acid, ascorbic acid, and stearic acid; and examples of the amine salt and the amino acid salt include a salt of an amine such as triethanolamine, and a salt of an amino acid such as glutamic acid. In addition, as others, salts of hyaluronic acid and chondroitin sulfuric acid, etc., aluminum zirconium glycine complex, etc., and further an acid-alkali neutralizing salt used in cosmetic formulation, etc., can also be used.

Examples of the antioxidant include tocopherol, p-t-butylphenol, butylhydroxyanisole, dibutylhydroxytoluene, and phytic acid; and examples of the pH adjuster include lactic acid, citric acid, glycolic acid, succinic acid, tartaric acid, dl-malic acid, potassium carbonate, sodium hydrogen carbonate, and ammonium hydrogen carbonate; and examples of the chelating agent include alanine, sodium edetate, sodium polyphosphate, sodium metaphosphate, and phosphoric acid; and examples of the refrigerant include L-menthol and camphor; and examples of the anti-inflammatory agent include allantoin, glycyrrhizic acid and a salt thereof, glycyrrhetinic acid and stearyl glycyrrhetinate, tranexamic acid, and azulene.

Examples of the skin beautifying component include a whitening agent such as a placenta extract, arbutin, glutathione, and a saxifrage extract; a cell activator or a rough skin ameliorating agent such as a royal jelly, a photosensitizer, a cholesterol derivative, and a bovine blood extract; a blood circulation promoter such as nonylic acid vanillylamide, benzyl nicotinate, β-butoxy¬ethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthin, and γ-oryzanol; a skin astringent such as zinc oxide and tannic acid; and an antiseborrheic drug such as sulfur and thiantrol.

Examples of the vitamins include vitamin A such as vitamin A oil, retinol, retinol acetate, and retinol palmitate; vitamin B2 such as riboflavin, riboflavin butyrate, and flavin adenine nucleotide; vitamin B6 such as pyridoxine hydrochloride, pyridoxine dioctanoate, and pyridoxine tripalmitate; vitamin B such as vitamin B12 and a derivative thereof, and vitamin B15 and a derivative thereof; vitamin C such as L-ascorbic acid, L-ascorbic acid dipalmitate, L-ascorbic acid-2-sulfate sodium, and L-ascorbic acid phosphate diester dipotassium; vitamin D such as ergocalciferol and cholecalciferol; vitamin E such as α-tocopherol, β-tocopherol, γ-tocopherol, dl-α-tocopherol acetate, dl-α-tocopherol nicotinate, and dl-α-tocopherol succinate; vitamin H; vitamin P; nicotinic acids such as nicotinic acid, benzyl nicotinate, and nicotinic acid amide; pantothenic acids such as calcium pantothenate, D-pantothenyl alcohol, pantothenyl ethyl ether, and acetyl pantothenyl ethyl ether; and biotin.

Examples of the amino acids include glycine, valine, leucine, isoleucine, serine, threonine, phenylalanine, arginine, lysine, aspartic acid, glutamic acid, cystine, cysteine, methionine, and tryptophan; and example of the nucleic acid includes deoxyribonucleic acid; and examples of the hormone include estradiol and ethenyl estradiol. Example of the inclusion compound includes cyclodextrin.

Examples of the solidifying agent for hair include amphoteric, anionic, cationic, and nonionic polymer compounds, and include a polyvinylpyrrolidone-based polymer compound such as polyvinyl-pyrrolidone and a vinylpyrrolidone/vinyl acetate copolymer; an acidic vinyl ether-based polymer compound such as a methyl vinyl ether/maleic anhydride alkyl half ester copolymer; an acidic polyvinyl acetate-based polymer such as a vinyl acetate/crotonic acid copolymer; an acidic acrylic-based polymer compound such as a (meth)acrylic acid/alkyl (meth)acrylate copolymer and a (meth)acrylic acid/alkyl (meth)acrylate/alkyl acrylamide copolymer; and an amphoteric acrylic-based polymer compound such as an N-methacryloylethyl-N,N-dimethyl-ammonium·α-N-methylcarboxybetaine/alkyl (meth)acrylate copolymer and a hydroxypropyl (meth)acrylate/butylaminoethyl methacrylate/octylamide acrylate copolymer. In addition, a naturally-derived polymer compound such as cellulose or a derivative thereof, and keratin and collagen or a derivative thereof can also be suitably used.

Examples of the form of the cosmetic in the present invention include a form such as a powdered, an oily, a water-in-oil emulsion, an oil-in-water emulsion, a non-aqueous emulsion, and a multi emulsion such as W/O/W and O/W/O.

In the present invention, examples of cosmetics include a skin care cosmetic such as a skin lotion, a milky lotion, a cream, a cleansing, a pack, an oil liquid, a massage agent, a beauty essence, a beauty oil, a detergent, a deodorant, a hand cream, a lip cream, and a wrinkle concealment; a makeup cosmetic such as a makeup base, a concealer, a white powder, a powder foundation, a liquid foundation, a cream foundation, an oil foundation, a blusher, an eye shadow, a mascara, an eyeliner, an eyebrow, and a lipstick; a hair cosmetic such as a shampoo, a rinse, a treatment, and a setting agent; an antiperspirant; and an ultraviolet protective cosmetic such as a sunscreen oil and a sunscreen milky lotion, and a sunscreen cream.

In addition, as the form of these cosmetics, various forms such as liquid, milky, cream, solid, paste, gel, powdered, pressed, multi-layered, mousse, spray, stick, and pencil can be selected.

### EXAMPLE

Hereinafter, the present invention will be described in more detail by way of Examples, but the present invention is not limited thereby. Incidentally, unless otherwise indicated, "%" described below means "mass %".

### (Example 1)

In a reactor were charged 100 parts by mass of the organohydrogenpolysiloxane represented by the following average composition formula (6), 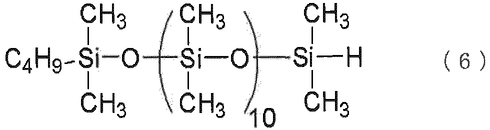 11.3 parts by mass of allyl urea, and 50 parts by mass of isopropyl alcohol, then 0.1 parts of a toluene solution containing 0.5% by mass of chloroplatinic acid was added to the mixture, and then the resulting mixture was reacted under reflux of the solvent for 3 hours. The reactant was heated under reduced pressure to distill off the solvent, whereby the following waxy organopolysiloxane was obtained by a yield of 96%. An IR and ¹H-NMR of the organopolysiloxane were measured to confirm that it was the urea group-containing organopolysiloxane represented by the following average composition formula (7).

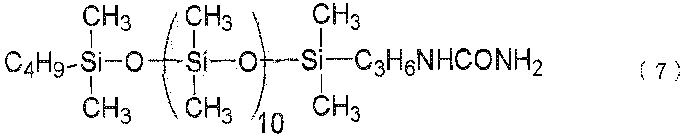

IR: 3346cm⁻¹(N-H), 2962cm⁻¹(C-H), 1650cm⁻¹(C=O), 1570cm⁻¹(N-H), 1260cm⁻¹(Si-CH₃), 1100-1020cm⁻¹(Si-O) ¹H-NMR(CDCl₃): 0ppm(s, 72H, Si-CH₃), 0.5ppm(t, 4H, Si-CH₂), 0.9ppm(t, 3H, C-CH₃), 1.3ppm(m, 4H, C-CH₂), 1.4ppm(m, 2H, C-CH₂), 2.1ppm(m, 2H, CH-N)

### (Example 2)

In a reactor were charged 100 parts by mass of the organohydrogenpolysiloxane represented by the following average composition formula (8), 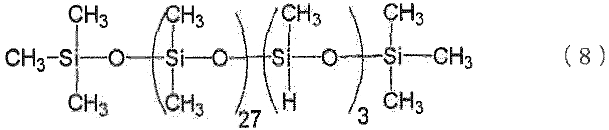 14.1 parts by mass of allyl urea, and 50 parts by mass of isopropyl alcohol, then 0.1 parts of a toluene solution containing 0.5% by mass of chloroplatinic acid was added to the mixture, and then the resulting mixture was reacted under reflux of the solvent for 3 hours. The reactant was heated under reduced pressure to distill off the solvent, whereby the following viscous liquid organopolysiloxane was obtained by a yield of 95%. An IR and ¹H-NMR of the organopolysiloxane were measured in the same manner as Example 1 to confirm that it was the urea group-containing organopolysiloxane represented by the following average composition formula (9).

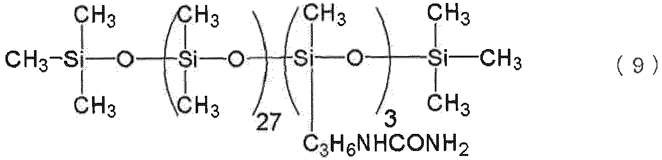

### (Example 3)

In a reactor were charged 100 parts by mass of the organohydrogenpolysiloxane represented by the following average composition formula (10), 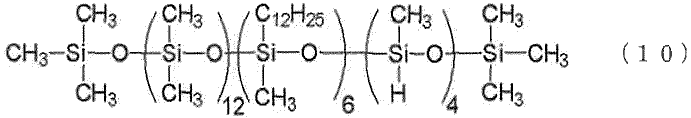 13.2 parts by mass of vinyl urea, and 50 parts by mass of isopropyl alcohol, then 0.1 parts by mass of a toluene solution containing 0.5% by mass of chloroplatinic acid was added to the mixture, and then the resulting mixture was reacted under reflux of the solvent for 3 hours. The reactant was heated under reduced pressure to distill off the solvent, whereby the following viscous liquid organopolysiloxane was obtained by a yield of 95%. An IR and ¹H-NMR of the organopolysiloxane were measured in the same manner as Example 1 to confirm that it was the urea group-containing organopolysiloxane represented by the following average composition formula (11).

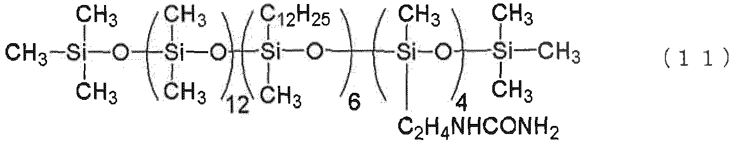

### (Example 4)

In a reactor were charged 100 parts by mass of the organohydrogenpolysiloxane represented by the following average composition formula (12), 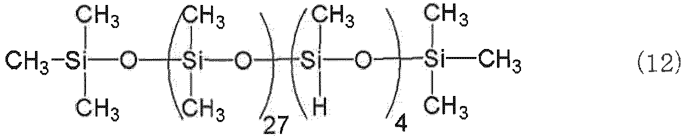 8.3 parts by mass of allyl urea, 70 parts by mass of the organopolysiloxane (13) having a vinyl group at one terminal represented by the following general formula, 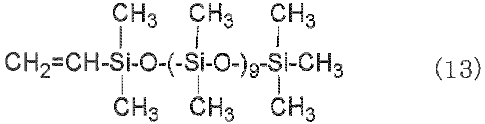 and 50 parts by mass of isopropyl alcohol, then 0.15 parts by mass of a toluene solution containing 0.5% by mass of chloroplatinic acid was added to the mixture, and then the resulting mixture was reacted under reflux of the solvent for 3 hours. The reactant was heated under reduced pressure to distill off the solvent, whereby the following viscous liquid organopolysiloxane was obtained by a yield of 94%. An IR and ¹H-NMR of the organopolysiloxane were measured in the same manner as Example 1 to confirm that it was the urea group-containing organopolysiloxane represented by the following average composition formula (14).

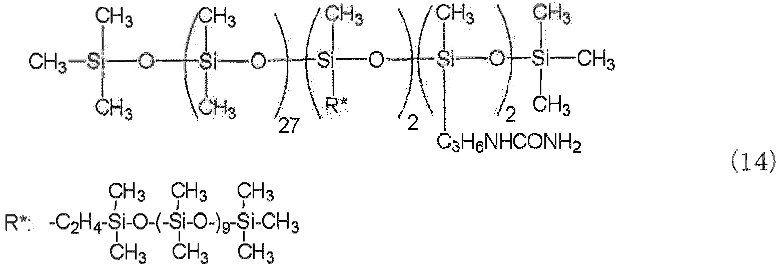

### (Example 5)

To a reactor were added 100 parts by mass of the amino group-containing organopolysiloxane represented by the following average composition (15), 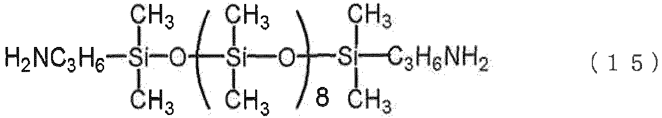 and 14.3 parts of urea, and then the mixture was reacted at 130°C for 3 hours. The residual ammonia gas was distilled off under reduced pressure, whereby the following waxy organopolysiloxane was obtained by a yield of 95%. An IR and ¹H-NMR of the organopolysiloxane were measured in the same manner as Example 1 to confirm that it was the urea group-containing organopolysiloxane represented by the following average composition formula (16).

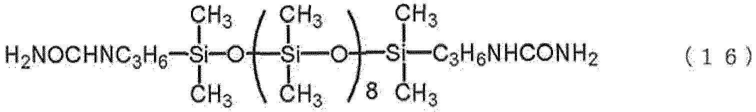

### (Examples 6, 7, and Comparative Example 1)

According to the preparation method shown below, emulsion cream foundations of the compositions shown in Table 1 was prepared.

**[Table 1]**

| Numbers | Composition (mass %) | Examples | | Comparative Example |
|---|---|---|---|---|
| | | 6 | 7 | 1 |
| 1 | Crosslinked polyether-modified silicone (Note 1) | 5.0 | 5.0 | 5.0 |
| 2 | Crosslinked dimethylpolysiloxane (Note 2) | 6.0 | 6.0 | 6.0 |
| 3 | Polyether-modified silicone (Note 3) | 1.0 | 1.0 | 1.0 |
| 4 | Dimethylpolysiloxane (Note 4) | 5.0 | 5.0 | 8.0 |
| 5 | Decamethylcyclopentasiloxane | 5.3 | 5.3 | 5.3 |
| 6 | Organopolysiloxane of Example 1 | 3.0 | - | - |
| 7 | Organopolysiloxane of Example 4 | - | 3.0 | - |
| 8 | Triethylhexanoin | 4.0 | 4.0 | 4.0 |
| 9 | Neopentyl glycol dioctanoate | 2.0 | 2.0 | 2.0 |
| 10 | Polymethylsilsesquioxane powder (Note 5) | 1.5 | 1.5 | 1.5 |
| 11 | 1,3-BG | 5.0 | 5.0 | 5.0 |
| 12 | Sodium chloride | 0.5 | 0.5 | 0.5 |
| 13 | Water | 50.0 | 50.0 | 50.0 |
| 14 | Silicone-treated titanium oxide (Note 6) | 8.65 | 8.65 | 8.65 |
| 15 | Silicone-treated red iron oxide (Note 6) | 0.45 | 0.45 | 0.45 |
| 16 | Silicone-treated yellow iron oxide (Note 6) | 0.75 | 0.75 | 0.75 |
| 17 | Silicone-treated black iron oxide (Note 6) | 0.15 | 0.15 | 0.15 |
| 18 | Antioxidant | 0.5 | 0.5 | 0.5 |
| 29 | Antiseptic | 1.0 | 1.0 | 1.0 |
| 20 | Perfume | 0.2 | 0.2 | 0.2 |
| Total | | 100 | 100 | 100 |

| | | | | |
|---|---|---|---|---|
| (Note 1) KSG-210: manufactured by Shin-Etsu Chemical Co., Ltd. (Note 2) KSG-15: manufactured by Shin-Etsu Chemical Co., Ltd. (Note 3) KF-6017: manufactured by Shin-Etsu Chemical Co., Ltd. (Note 4) KF-96A-6cs: manufactured by Shin-Etsu Chemical Co., Ltd. (Note 5) KMP-590: manufactured by Shin-Etsu Chemical Co., Ltd. (Note 6) KF-9909: manufactured by Shin-Etsu Chemical Co., Ltd., treatment | | | | |

### <Preparation of cosmetics>

The Components (1) to (4), part of the component (5), the components (6) to (10) and (18), (19) were stirred and mixed to be homogeneous. The components (11) and (12) dissolved separately and uniformly in the component (13) were gently added to the mixture and emulsified. The components (14) to (17), the remainder of the component (5), and the component (20) were added to the emulsion and mixed. The mixture was filled in a prescribed container to prepare an emulsion cream foundation. The following evaluations were conducted on the resulting emulsion cream foundations.

### [Usability evaluation]

Spreading at the time of application (spreadability), sticky feeling, finished color unevenness, cosmetic durability (durability (evaluation 8 hours after application)) of the resulting emulsion cream foundations were evaluated by 50 female expert panelists according to the following criteria and averaged the evaluation results.

### [Secondary adhesion prevention effect]

The emulsion cream foundations were applied to the foreheads of the expert panelists respectively by the same operation, and at 20 minutes after the application, tissue paper was pressed against the applied part, and the secondary adhesion prevention effect of the cosmetic was evaluated according to the following criteria. The average of the evaluation results was taken.

**[Table 2]**

| Points | Spread-ability | Sticky feeling | Color unevenness | Cosmetic durability | Secondary adhesion |
|---|---|---|---|---|---|
| 5 | Good | None | None | Good | None |
| 4 | Slightly good | Nearly none | Nearly none | Slightly good | Nearly none |
| 3 | Normal | Normal | Normal | Normal | Normal |
| 2 | Slightly bad | Slightly sticky | Slightly uneven | Slightly bad | Slightly bad |
| 1 | Bad | Sticky | Considerable uneven | Bad | Bad |

The results are summarized based on the following and shown in Table 3 based on the average points of the obtained measurement results.
⊚: The average point is 4.0 points or more
○: The average point is 3.0 points or more and less than 4.0 points
Δ: The average point is 2.0 points or more and less than 3.0 points
×: The average point is less than 2.0 points

**[Table 3]**

| Items | Example 6 | Example 7 | Comparative Example 1 |
|---|---|---|---|
| Spreadability | ⊚ | ⊚ | ⊚ |
| Sticky feeling | ⊚ | ⊚ | ⊚ |
| Color unevenness | ⊚ | ⊚ | ○ |
| Cosmetic durability | ⊚ | ⊚ | Δ |
| Secondary adhesion | ⊚ | ⊚ | Δ |

As evident from Table 3, the cosmetics of the present invention (Examples 6 and 7) is significantly superior to Comparative Example 1 in the cosmetic durability and the secondary adhesion prevention, and also has good usability.

### (Examples 8, 9, and Comparative Example 2)

Lipsticks of the compositions shown in Table 4 was prepared.

**[Table 4]**

| Numbers | Composition (mass %) | Examples | | Comparative Example |
|---|---|---|---|---|
| | | 8 | 9 | 2 |
| 1 | Candelilla wax | 6.0 | 6.0 | 6.0 |
| 2 | Polyethylene | 2.0 | 2.0 | 2.0 |
| 3 | Microcrystalline wax | 4.0 | 4.0 | 4.0 |
| 4 | Ceresin | 8.0 | 8.0 | 8.0 |
| 5 | Stearyl-modified acrylic silicone resin (Note 1) | 12.0 | 12.0 | 12.0 |
| 6 | Diphenyl dimethicone (Note 2) | 17.8 | 17.8 | 17.8 |
| 7 | Organopolysiloxane of Example 2 | 4.0 | | |
| 8 | Organopolysiloxane of Example 3 | | 4.0 | |
| 9 | Dimethylpolysiloxane (20 mm²/s) | | | 4.0 |
| 10 | Alkyl-modified branched polyglycerol-modified silicone (Note 3) | 3.0 | 3.0 | 3.0 |
| 11 | Macadamia nut oil | 15.0 | 15.0 | 15.0 |
| 12 | Hydrogenated polyisobutene | 8.0 | 8.0 | 8.0 |
| 13 | Isotridecyl isononanoate | 5.0 | 5.0 | 5.0 |
| 14 | Perfume | 0.2 | 0.2 | 0.2 |
| 15 | Lipstick pigment | 10 | 10 | 10 |
| 16 | Mica | 5 | 5 | 5 |
| Total | | 100 | 100 | 100 |

| | | | | |
|---|---|---|---|---|
| (Note 1) KP-561P: manufactured by Shin-Etsu Chemical Co., Ltd. (Note 2) KF-54: manufactured by Shin-Etsu Chemical Co., Ltd. (Note 3) KF-6105: manufactured by Shin-Etsu Chemical Co., Ltd. | | | | |

### <Preparation of cosmetics>

A: The components 1 to 12, 15 and 16 were heated and uniformly mixed.
B: Under heating, the components 13 and 14 were added to A and uniformly mixed, the mixture was filled in a highly airtight prescribed container to obtain a lipstick.

### [Usability evaluation]

Spreading at the time of application (spreadability), sticky feeling, finished color unevenness, cosmetic durability (durability (evaluation 8 hours after application)) of the resulting lipsticks were evaluated by 50 female expert panelists in the same manner as Examples 6 and 7 and averaged the evaluation results.

**[Table 5]**

| Items | Example 8 | Example 9 | Comparative Example 2 |
|---|---|---|---|
| Spreadability | ⊚ | ⊚ | ⊚ |
| Sticky feeling | ⊚ | ⊚ | ○ |
| Color unevenness | ⊚ | ⊚ | Δ |
| Cosmetic durability | ⊚ | ⊚ | Δ |

It was confirmed that the lipsticks obtained as described above was not sticky and not oily, also free from bleeding, etc., and was also good in the cosmetic durability.

Hereinafter, formulation examples of cosmetics will be shown. Hereinafter, "spreadability", "cosmetic durability" were evaluated according to the same criteria as described above, and with regard to foundations, the secondary adhesion prevention was also evaluated according to the same criteria as described above.

### (Example 10) powder foundation

| Composition | Mass (%) |
|---|---|
| 1. Silicone-treated titanium oxide (Note 1) | 12.0 |
| 2. Silicone-treated sericite (Note 1) | 35.0 |
| 3. Lecithin-treated talc | 35.1 |
| 4. Lecithin-treated spherical nylon powder | 5.0 |
| 5. Silicone-treated red oxide (Note 1) | 0.4 |
| 6. Silicone-treated yellow iron oxide (Note 1) | 2.0 |
| 7. Silicone-treated umber (Note 1) | 0.4 |
| 8. Silicone-treated black iron oxide (Note 1) | 0.1 |
| 9. Organopolysiloxane of Example 1 | 3.0 |
| 10. Crosslinked dimethylpolysiloxane (Note 2) | 4.0 |
| 11. Glyceryl trioctanoate | 1.5 |
| 12. Silicone wax (Note 3) | 1.5 |
| Total | 100.0 |

| | |
|---|---|
| (Note 1) KF-9909: manufactured by Shin-Etsu Chemical Co., Ltd., treatment (Note 2) KSG-16: manufactured by Shin-Etsu Chemical Co., Ltd. (Note 3) KP-562P: manufactured by Shin-Etsu Chemical Co., Ltd. | |

### <Preparation of cosmetics>

A: The components 1 to 8 were mixed, and the mixture was uniformly ground.
B: The components 9 to 12 were uniformly mixed.
C: B was added to A and homogenized, and the mixture was pressed into a mold to obtain a powder foundation.

The resulting powder foundation spread lightly, had good cosmetic durability and no secondary adhesion.

### (Example 11) powder foundation

| Composition | Mass (%) |
|---|---|
| 1. Caprylylsilane-treated mica (Note 1) | 40.0 |
| 2. Silicone-treated talc (Note 2) | 20.0 |
| 3. Silicone-treated titanium oxide (Note 2) | 8.0 |
| 4. Silicone-treated fine particle titanium oxide (Note 2) | 5.0 |
| 5. Silicone-treated barium sulfate (Note 2) | 8.9 |
| 6. Silicone-treated foundation pigment (Note 2) | 7.0 |
| 7. Phenyl-modified hybrid silicone composite powder (Note 3) | 2.0 |
| 8. Polymethylsilsesquioxane powder (Note 4) | 0.4 |
| 9. Antiseptic | 0.5 |
| 10. Perfume | 0.2 |
| 11. Organopolysiloxane of Example 2 | 3.0 |
| 12. Glyceryl trioctanoate | 3.0 |
| 13. Squalane | 1.0 |
| 14. Vaseline | 1.0 |
| Total | 100.0 |

| | |
|---|---|
| (Note 1) AES-3083: manufactured by Shin-Etsu Chemical Co., Ltd., treatment (Note 2) KF-9909: manufactured by Shin-Etsu Chemical Co., Ltd., treatment (Note 3) KSP-300: manufactured by Shin-Etsu Chemical Co., Ltd. (Note 4) KMP-590: manufactured by Shin-Etsu Chemical Co., Ltd. | |

### <Preparation of cosmetics>

A: The components 1 to 9 were mixed, and the mixture was uniformly ground.
B: The components 11 to 14 were uniformly mixed, and the mixture was added to A to make them uniform.
C: The component 10 was added to B, and the mixture was pressed into a mold to obtain a powder foundation.

The resulting powder foundation spread lightly, had excellent cosmetic durability and no secondary adhesion.

### (Example 12) stick W/O foundation

| Composition | Mass (%) |
|---|---|
| 1. Ceresin | 5.5 |
| 2. Inulin stearate | 2.0 |
| 3. Neopentyl glycol dioctanoate | 7.0 |
| 4. Triethylhexanoin | 4.0 |
| 5. Dimethylpolysiloxane (6cs) | 6.3 |
| 6. Organopolysiloxane of Example 5 | 3.0 |
| 7. Crosslinked polyglycerol-modified silicone (Note 1) | 4.0 |
| 8. Alkyl-modified branched polyglycerol-modified silicone (Note 2) | 1.5 |
| 9. Polymethylsilsesquioxane powder (Note 3) | 1.0 |
| 10. Silicone-treated titanium oxide (Note 4) | 9.0 |
| 11. Silicone-treated foundation pigment (Note 5) | 5.0 |
| 12. Lecithin | 0.2 |
| 13. Polysorbate 80 | 0.3 |
| 14. 1,3-BG | 4.0 |
| 15. Antiseptic | 0.5 |
| 16. Perfume | 0.2 |
| 17. Purified water | 46.5 |
| Total | 100.0 |

| | |
|---|---|
| (Note 1) KSG-710: manufactured by Shin-Etsu Chemical Co., Ltd. (Note 2) KF-6105: manufactured by Shin-Etsu Chemical Co., Ltd. (Note 3) KMP-590: manufactured by Shin-Etsu Chemical Co., Ltd. (Note 4) KF-9909: manufactured by Shin-Etsu Chemical Co., Ltd., treatment (Note 5) KF-9909: manufactured by Shin-Etsu Chemical Co., Ltd., treatment | |

### <Preparation of cosmetics>

A: The components 1 to 9 were heated, dissolved, and homogenized.
B: The components 10 to 13 and part of the component 14 were mixed, and the mixture was dispersed by a roller.
C: The remaining of the component 14, and the components 15 and 17 were uniformly dissolved, the solution was added to B, and under heating, the mixture was uniformly dispersed.
D: Under heating and stirring, C was added to A and emulsified, the component 16 was added to the emulsion, and the mixture was filled in a highly airtight prescribed container to obtain a stick W/O foundation.

The resulting stick W/O foundation spread lightly, had excellent cosmetic durability and no secondary adhesion.

### (Example 13) polyhydric alcohol-in-oil emulsion solid rouge

| Composition | Mass (%) |
|---|---|
| 1. Crosslinked polyglycerol-modified silicone (Note 1) | 5.0 |
| 2. Crosslinked dimethylpolysiloxane (Note 2) | 5.0 |
| 3. Decamethylcyclopentasiloxane | 5.0 |
| 4. Dimethylpolysiloxane (6cs) | 19.7 |
| 5. Cetyl isooctanoate | 8.0 |
| 6. Organopolysiloxane of Example 1 | 5.0 |
| 7. Behenyl-modified acrylic silicone resin (Note 3) | 3.0 |
| 8. Paraffin wax (Melting point 80°C) | 9.0 |
| 9. Dimethyl distearyl ammonium hectorite | 0.3 |
| 10. Acrylic silicone-treated powder (Note 4) | 25.0 |
| 11. Antiseptic | 0.5 |
| 12. Perfume | 0.2 |
| 13. 1,3-butylene glycol | 14.3 |
| Total | 100.0 |

| | |
|---|---|
| (Note 1) KSG-710: manufactured by Shin-Etsu Chemical Co., Ltd. (Note 2) KSG-15: manufactured by Shin-Etsu Chemical Co., Ltd. (Note 3) KP-562P: manufactured by Shin-Etsu Chemical Co., Ltd. (Note 4) KP-574: manufactured by Shin-Etsu Chemical Co., Ltd., treatment | |

### <Preparation of cosmetics>

A: The components 1 to 9 and 12 were heated to 80°C and uniformly mixed.
B: The component 10 was added to A and uniformly dispersed.
C: The components 11 and 13 were mixed and heated to 80°C, and then the mixture was added to B and emulsified, poured into a gold plate and cooled to obtain a solid rouge.

The polyhydric alcohol-in-oil emulsion solid rouge obtained as described above was light in the spreadability, and also not sticky and not oily.

### (Example 14) cream lipstick

| Composition | Mass (%) |
|---|---|
| 1. Palmitic acid / ethyl hexanoic acid dextrin (Note 1) | 9.0 |
| 2. Triethylhexanoin | 9.0 |
| 3. Organopolysiloxane of Example 1 | 5.0 |
| 4. Alkyl-modified crosslinked dimethylpolysiloxane (Note 2) | 8.0 |
| 5. Alkyl-modified branched polyglycerol-modified silicone (Note 3) | 2.0 |
| 6. Decamethylcyclopentasiloxane | 36.0 |
| 7. 1,3-butylene glycol | 4.8 |
| 8. Purified water | 18.0 |
| 9. Coloring pigment | 6.0 |
| 10. Mica | 2.0 |
| 11. Perfume | 0.2 |
| Total | 100.0 |

| | |
|---|---|
| (Note 1) Rheopearl TT: manufactured by Chiba Flour Milling Co.,Ltd. (Note 2) KSG-43: manufactured by Shin-Etsu Chemical Co., Ltd. (Note 3) KF-6105: manufactured by Shin-Etsu Chemical Co., Ltd. | |

### <Preparation of cosmetics>

A: The component 1, part of the component 2, and the components 3 to 6 were heated and uniformly mixed.
B: The component 9 was mixed with the remainder of the component 2, and dispersed by a roller, the dispersion was added to A and uniformly mixed.
C: The components 7 and 8 were mixed, heated, and added to B, and the mixture was emulsified.
D: The components 10 and 11 were added to C to obtain a cream lipstick.

The cream lipstick obtained as described above was light in the spreadability, easy to spread on the lip, also not sticky and not oily, and also good in the durability.

### (Example 15) cream eye shadow

| Composition | Mass (%) |
|---|---|
| 1. Decamethylcyclopentasiloxane | 15.0 |
| 2. Dimethylpolysiloxane (6cs) | 6.0 |
| 3. Organopolysiloxane of Example 5 | 3.0 |
| 4. Branched polyether-modified silicone (Note 1) | 1.5 |
| 5. Acrylic silicone resin-treated pigment (Note 2) | 16.0 |
| 6. Sodium chloride | 2.0 |
| 7. Propylene glycol | 7.5 |
| 8. Antiseptic | 0.5 |
| 9. Purified water | 48.5 |
| Total | 100.0 |

| | |
|---|---|
| (Note 1) KF-6028P: manufactured by Shin-Etsu Chemical Co., Ltd. (Note 2) KP-574: manufactured by Shin-Etsu Chemical Co., Ltd., treatment | |

### <Preparation of cosmetics>

A: The components 1 to 4 were mixed, and the component 5 was added to the mixture, and uniformly mixed and dispersed.
B: The components 6 to 9 were mixed.
C: B was added to A and emulsified to obtain a cream eye shadow.

The cream eye shadow obtained as described above was light in the spreadability, not oily and not powdery, and also good in the durability.

### (Example 16) cream eye shadow

| Composition | Mass (%) |
|---|---|
| 1. Acrylic silicone resin dissolved material (Note 1) | 5.0 |
| 2. Stearyl-modified acrylic silicone resin (Note 2) | 2.0 |
| 3. Branched polyether-modified silicone (Note 3) | 1.5 |
| 4. Decamethylcyclopentasiloxane | 23.3 |
| 5. Organopolysiloxane of Example 3 | 5.0 |
| 6. Dimethyl distearyl ammonium hectorite | 1.2 |
| 7. Acrylic silicone resin-treated pigment (Note 4) | 20.0 |
| 8. Spherical nylon | 3.0 |
| 9. Talc | 4.0 |
| 10. Ethanol | 5.0 |
| 11. Purified water | 30.0 |
| Total | 100.0 |

| | |
|---|---|
| (Note 1) KP-545: manufactured by Shin-Etsu Chemical Co., Ltd. (Note 2) KP-561P: manufactured by Shin-Etsu Chemical Co., Ltd. (Note 3) KF-6028P: manufactured by Shin-Etsu Chemical Co., Ltd. (Note 4) KP-574: manufactured by Shin-Etsu Chemical Co., Ltd., treatment | |

### <Preparation of cosmetics>

A: The components 1 to 6 were mixed, and the components 7 to 9 was added to the mixture, and uniformly mixed and dispersed.
B: The components 10 to 11 were mixed.
C: B was added to A and emulsified to obtain a cream eye shadow.

The cream eye shadow obtained as described above was light in the spreadability, not oily and not powdery, was fresh, and also good in the durability.

### (Example 17) sun-cut milky lotion

| Composition | Mass (%) |
|---|---|
| 1. Crosslinked polyether-modified silicone (Note 1) | 3.0 |
| 2. Crosslinked dimethylpolysiloxane (Note 2) | 2.0 |
| 3. Branched polyether-modified silicone (Note 3) | 1.0 |
| 4. Organopolysiloxane of Example 2 | 2.0 |
| 5. Decamethylcyclopentasiloxane | 6.0 |
| 6. Isotridecyl isononanoate | 6.0 |
| 7. Titanium oxide dispersion (Note 4) | 25.0 |
| 8. Zinc oxide dispersion (Note 5) | 35.0 |
| 9. 1,3-butylene glycol | 2.0 |
| 10. Sodium citrate | 0.2 |
| 11. Sodium chloride | 0.5 |
| 12. Purified water | 17.3 |
| Total | 100.0 |

| | |
|---|---|
| (Note 1) KSG-210: manufactured by Shin-Etsu Chemical Co., Ltd. (Note 2) KSG-15: manufactured by Shin-Etsu Chemical Co., Ltd. (Note 3) KF-6028P: manufactured by Shin-Etsu Chemical Co., Ltd. (Note 4) SPD-T5: manufactured by Shin-Etsu Chemical Co., Ltd. (Note 5) SPD-Z5: manufactured by Shin-Etsu Chemical Co., Ltd. | |

### <Preparation of cosmetics>

A: The components 1 to 6 were uniformly mixed.
B: The components 9 to 12 were mixed.
C: B was added to A and emulsified, the components 7 and 8 were added to the emulsion to obtain a sun-cut milky lotion.

The sun-cut milky lotion obtained as described above was light in the spreadability, not sticky and not oily, and good in perspiration resistance.

### (Example 18) sun-cut cream

| Composition | Mass (%) |
|---|---|
| 1. Crosslinked polyether-modified silicone (Note 1) | 3.0 |
| 2. Crosslinked dimethylpolysiloxane (Note 2) | 2.0 |
| 3. Alkyl-modified branched polyether-modified silicone (Note 3) | 1.0 |
| 4. Organopolysiloxane of Example 1 | 5.0 |
| 5. Decamethylcyclopentasiloxane | 17.5 |
| 6. Octyl methoxycinnamate | 6.0 |
| 7. Acrylic silicone resin dissolved material (Note 4) | 10.0 |
| 8. Lipophilizing-treated fine particle zinc oxide (Note 5) | 20.0 |
| 9. 1,3-butylene glycol | 1.8 |
| 10. Sodium citrate | 0.2 |
| 11. Sodium chloride | 0.5 |
| 12. Perfume | 0.2 |
| 13. Purified water | 32.8 |
| Total | 100.0 |

| | |
|---|---|
| (Note 1) KSG-240: manufactured by Shin-Etsu Chemical Co., Ltd. (Note 2) KSG-15: manufactured by Shin-Etsu Chemical Co., Ltd. (Note 3) KF-6038: manufactured by Shin-Etsu Chemical Co., Ltd. (Note 4) KP-575: manufactured by Shin-Etsu Chemical Co., Ltd. (Note 5) AES-3083: manufactured by Shin-Etsu Chemical Co., Ltd., treatment | |

### <Preparation of cosmetics>

A: The component 7 was added to part of the component 5 and homogenized, and the component 8 was added to the mixture and dispersed by a beads mill.
B: The components 1 to 4 and the remainder of the component 5, and the component 6 were uniformly mixed.
C: The components 9 to 11 and the component 13 were mixed, and homogenized.
D: C was added to B and emulsified, A and the component 12 were added to the emulsion to obtain a sun-cut cream.

The sun-cut cream obtained as described above was not sticky, was light in the spreadability, not oily, gave a refreshing feeling in use, and was also good in the durability.

### (Example 19) Sun-cut lotion (shaking type)

| Composition | Mass (%) |
|---|---|
| 1. Branched polyether-modified silicone (Note 1) | 2.0 |
| 2. Organopolysiloxane of Example 1 | 3.0 |
| 3. Dimethylpolysiloxane (6cs) | 4.0 |
| 4. Decamethylcyclopentasiloxane | 8.8 |
| 5. Ethyl hexyl methoxycinnamate | 7.5 |
| 6. Hybrid silicone composite powder (Note 2) | 0.5 |
| 7. Dimethyl distearyl ammonium hectorite | 0.2 |
| 8. Zinc oxide dispersion (Note 3) | 45.0 |
| 9. 1,3-butylene glycol | 3.0 |
| 10. Alcohol | 5.0 |
| 11. Sodium citrate | 0.2 |
| 12. Sodium chloride | 0.5 |
| 13. Purified water | 20.3 |
| Total | 100.0 |

| | |
|---|---|
| (Note 1) KF-6028P: manufactured by Shin-Etsu Chemical Co., Ltd. (Note 2) KSP-105: manufactured by Shin-Etsu Chemical Co., Ltd. (Note 3) SPD-Z6: manufactured by Shin-Etsu Chemical Co., Ltd. | |

### <Preparation of cosmetics>

A: The components 1 to 7 were uniformly mixed.
B: The components 9 to 13 were mixed.
C: B was added to A and emulsified, the component 8 was added to the emulsion to obtain a shaking type sun-cut lotion.

The sun-cut lotion obtained as described above was light in the spreadability, not sticky and not oily, and was also very excellent in the durability.

### (Example 20) suntan cream

| Composition | Mass (%) |
|---|---|
| 1. Alkyl-modified crosslinked polyether-modified silicone (Note 1) | 4.0 |
| 2. Alkyl-modified crosslinked dimethylpolysiloxane (Note 2) | 2.0 |
| 3. Alkyl-modified branched polyether-modified silicone (Note 3) | 1.0 |
| 4. Organopolysiloxane of Example 5 | 3.0 |
| 5. Decamethylcyclopentasiloxane | 12.3 |
| 6. Stearyl-modified acrylic silicone (Note 4) | 1.0 |
| 7. Dimethyl octyl para aminobenzoic acid | 1.5 |
| 8. 4-t-Butyl-4'-methoxy-dibenzoylmethane | 1.5 |
| 9. Kaolin | 0.5 |
| 10. Pigment | 8.0 |
| 11. Titanium oxide coated mica | 8.0 |
| 12. Dioctadecyl dimethyl ammonium chloride | 0.1 |
| 13. Sodium L-glutamate | 3.0 |
| 14. 1,3-butylene glycol | 4.0 |
| 15. Sodium citrate | 0.2 |
| 16. Sodium chloride | 0.5 |
| 17. Antioxidant | 0.5 |
| 18. Antiseptic | 0.5 |
| 19. Perfume | 0.2 |
| 20. Purified water | 48.2 |
| Total | 100.0 |

| | |
|---|---|
| (Note 1) KSG-320: manufactured by Shin-Etsu Chemical Co., Ltd. (Note 2) KSG-42: manufactured by Shin-Etsu Chemical Co., Ltd. (Note 3) KF-6038: manufactured by Shin-Etsu Chemical Co., Ltd. (Note 4) KP-561P: manufactured by Shin-Etsu Chemical Co., Ltd. | |

### <Preparation of cosmetics>

A: The components 1 to 8, 17 and 18 were heated and mixed.
B: The component 12 and part of the component 20 were heated and stirred, and then the components 9 to 11 were added to the mixture and dispersed.
C: The components 13 to 16 and the remainder of the component 20 were uniformly dissolved, and mixed with B.
D: Under stirring, C was gradually added to A and emulsified, and cooled, and the component 19 was added to the emulsion to obtain a suntan cream.

The suntan cream obtained as described above had a fine texture, was light in the spreadability, not sticky and not oily, gave the refreshing feeling in use, and was also good in the durability.

### (Example 21) liquid W/O foundation

| Composition | | Mass (%) |
|---|---|---|
| 1. | Decamethylcyclopentasiloxane | 18.0 |
| 2. | Dimethylpolysiloxane (6cs) | 4.0 |
| 3. | Organopolysiloxane of Example 4 | 4.0 |
| 4. | Alkyl-modified branched polyether-modified silicone (Note 1) | 2.0 |
| 5. | Ethyl hexyl paramethoxycinnamate | 3.0 |
| 6. | Fluorine-modified silicone (Note 2) | 2.0 |
| 7. | Polymethylsilsesquioxane powder (Note 3) | 1.5 |
| 8. | Fluorine compound-treated foundation pigment (Note 4) | 9.3 |
| 9. | Fluorine compound-treated mica titanium (Note 4) | 2.0 |
| 10. | Silicone-treated fine particle titanium oxide (Note 5) | 8.0 |
| 11. | Alkyl-modified branched polyglycerol-modified | |
| | silicone (Note 6) | 1.2 |
| 12. | Ethanol | 4.0 |
| 13. | 1,3-butylene glycol | 4.3 |
| 14. | Glycerol | 1.5 |
| 15. | Magnesium sulfate | 0.5 |
| 16. | Antioxidant | 0.5 |
| 17. | Antiseptic | 0.5 |
| 18. | Perfume | 0.2 |
| 19. | Purified water | 33.5 |
| Total | | 100.0 |

| | | |
|---|---|---|
| (Note 1) KF-6038: manufactured by Shin-Etsu Chemical Co., Ltd. (Note 2) FL-5: manufactured by Shin-Etsu Chemical Co., Ltd. (Note 3) KMP-590: manufactured by Shin-Etsu Chemical Co., Ltd. (Note 4) 5% coated with perfluoroalkylethyl phosphate diethanolamine salt (Note 5) KF-9909: manufactured by Shin-Etsu Chemical Co., Ltd., treatment (Note 6) KF-6105: manufactured by Shin-Etsu Chemical Co., Ltd. | | |

### <Preparation of cosmetics>

A: Part of the component 1, the components 10 and 11 were mixed, and uniformly dispersed.
B: The components 7 to 9 were uniformly mixed.
C: The remainder of the component 1, the components 2 to 6 were mixed, and B was added to the mixture, and uniformly mixed and dispersed.
D: The components 12 to 17, and 19 were mixed, and homogenized.
E: Under stirring, D was gradually added to C and emulsified, and A and the component 18 was added to the emulsion to obtain a W/O foundation.

The W/O foundation obtained as described above was not sticky and was smooth, was light in the spreadability, not oily, and also good in the cosmetic durability, and also free from the secondary adhesion.

### (Example 22) hair cream

| Composition | | Mass (%) |
|---|---|---|
| 1. | Decamethylcyclopentasiloxane | 16.0 |
| 2. | Methylphenylpolysiloxane (Note 1) | 3.0 |
| 3. | Organopolysiloxane of Example 3 | 2.0 |
| 4. | Squalane | 6.0 |
| 5. | Silicone network resin dissolved material (Note 2) | 2.0 |
| 6. | Sorbitan sesquiisostearate | 1.5 |
| 7. | Alkyl-modified branched polyether-modified silicone (Note 3) | 2.0 |
| 8. | Sorbitol sodium sulfate | 2.0 |
| 9. | Chondroitin sodium sulfate | 1.0 |
| 10. | Sodium hyaluronate | 0.5 |
| 11. | Propylene glycol | 2.3 |
| 12. | Antiseptic | 1.5 |
| 13. | Vitamin E acetate | 0.1 |
| 14. | Antioxidant | 0.5 |
| 15. | Perfume | 0.2 |
| 16. | Purified water | 59.4 |
| Total | | 100.0 |

| | | |
|---|---|---|
| (Note 1) KF-54: manufactured by Shin-Etsu Chemical Co., Ltd. (Note 2) KF-7312J: manufactured by Shin-Etsu Chemical Co., Ltd. (Note 3) KF-6038: manufactured by Shin-Etsu Chemical Co., Ltd. | | |

### <Preparation of cosmetics>

A: The components 1 to 7, and the components 12 to 14 were uniformly mixed.
B: The components 8 to 11, and 16 were uniformly mixed.
C: Under stirring, B was gradually added to A and emulsified, and the component 15 was added to the emulsion to obtain a hair cream.

The hair cream obtained as described above was not oily, was light in the spreadability, was water resistant, water repellent, perspiration resistant, and also good in the durability.

### (Example 23) hair cream

| Composition | Mass (%) |
|---|---|
| 1. Silicone gum dissolved material (Note 1) | 10.0 |
| 2. Silicone network resin dissolved material (Note 2) | 10.0 |
| 3. Organopolysiloxane of Example 1 | 5.0 |
| 4. Glyceryl tri-2-ethylhexanoate | 10.0 |
| 5. Vaseline | 5.0 |
| 6. Stearic acid | 1.5 |
| 7. Cetyl alcohol | 0.5 |
| 8. Polyglyceryl monooleate | 1.5 |
| 9. Glyceryl monostearate | 1.5 |
| 10. Polyether-modified silicone (Note 3) | 0.5 |
| 11. 1,3-butylene glycol | 5.0 |
| 12. (Acrylates / alkyl acrylate (C10-30)) cross polymer (Note 4) | 0.3 |
| 13. Triethanolamine | 0.3 |
| 14. Antiseptic | 0.5 |
| 15. Perfume | 0.2 |
| 16. Purified water | 48.2 |
| Total | 100.0 |

| | |
|---|---|
| (Note 1) KF-9028: manufactured by Shin-Etsu Chemical Co., Ltd. (Note 2) KF-7312J: manufactured by Shin-Etsu Chemical Co., Ltd. (Note 3) KF-6011: manufactured by Shin-Etsu Chemical Co., Ltd. (Note 4) Pemulen TR-1: manufactured by Noveon International, Inc. | |

### <Preparation of cosmetics>

A: The components 1 to 10, and the component 14 were heated and dissolved.
B: The components 11 to 13, and 16 were mixed and heated.
C: Under stirring, A was gradually added to B and emulsified, and cooled, and then the component 15 was added to the emulsion to obtain a hair cream.

The hair cream obtained as described above was light in the spreadability, gave the hair gloss and smoothness, had an excellent setting effect on the hair, was water resistant, perspiration resistant, and also good in the durability.

### (Example 24) moisturizing O/W cream

| (Component) | Mass (%) |
|---|---|
| 1. Organopolysiloxane of Example 4 | 4.0 |
| 2. Liquid paraffin | 4.5 |
| 3. Macadamia nut oil | 5.0 |
| 4. Dimethylpolysiloxane (viscosity 6mm²/s:25°C) | 5.0 |
| 5. Octyl paramethoxycinnamate | 5.0 |
| 6. Alkyl-modified branched polyglycerol-modified silicone (Note 1) | 1.5 |
| 7. Propylene glycol | 8.0 |
| 8. Glycerol | 3.0 |
| 9. Antiseptic | 0.5 |
| 10. Perfume | 0.2 |
| 11. Purified water | 63.3 |
| Total | 100.0 |

| | |
|---|---|
| (Note 1) KF-6105: manufactured by Shin-Etsu Chemical Co., Ltd. | |

### <Preparation of cosmetics>

A: The components 1 to 6 were uniformly mixed.
B: The components 7 to 11 were mixed, and then added to A, and the mixture was emulsified.

The moisturizing O/W cream obtained as described above was light in the spreadability, gave the refreshing feeling in use, and maintained a moisturizing effect.

### (Example 25) O/W emollient cream

| Composition | Mass (%) |
|---|---|
| 1. Crosslinked dimethylpolysiloxane (Note 1) | 7.0 |
| 2. Crosslinked dimethylpolysiloxane (Note 2) | 30.0 |
| 3. Organopolysiloxane of Example 5 | 3.0 |
| 4. Decamethylcyclopentasiloxane | 6.0 |
| 5. 1,3-butylene glycol | 1.0 |
| 6. Branched polyglycerol-modified silicone (Note 3) | 0.6 |
| 7. Branched polyglycerol-modified silicone (Note 4) | 0.3 |
| 8. (Acrylamide / Acryloyl dimethyl taurine Na) copolymer (Note 5) | 0.6 |
| 9. Dimethyl taurine ammonium acrylate / VP copolymer (Note 6) | 0.7 |
| 10. Sodium chloride | 0.1 |
| 11. Purified water | 50.7 |
| Total | 100.0 |

| | |
|---|---|
| (Note 1) KSG-15: manufactured by Shin-Etsu Chemical Co., Ltd. (Note 2) KSG-16: manufactured by Shin-Etsu Chemical Co., Ltd. (Note 3) KF-6104: manufactured by Shin-Etsu Chemical Co., Ltd. (Note 4) KF-6100: manufactured by Shin-Etsu Chemical Co., Ltd. (Note 5) Simulgel 600: manufactured by Seppic S.A. (Note 6) Aristoflex AVC: manufactured by Clariant AG | |

### <Preparation of cosmetics>

A: The components 1 to 4 were uniformly mixed.
B: The components 5 to 11 were uniformly mixed.
C: Under stirring, A was gradually added to B and mixed to obtain a O/W emollient cream.

The O/W emollient cream obtained as described above was not oily and was smooth, was light in the spreadability, and maintained a skin protection effect.

### (Example 26) hand cream

| Composition | Mass (%) |
|---|---|
| 1. Decamethylcyclopentasiloxane | 30.0 |
| 2. Organopolysiloxane of Example 2 | 5.0 |
| 3. Liquid paraffin | 5.0 |
| 4. Amino-modified silicone gum dissolved material (Note 1) | 8.0 |
| 5. Branched polyether-modified silicone (Note 2) | 2.0 |
| 6. Hybrid silicone composite powder (Note 3) | 2.5 |
| 7. Distearyl dimethyl ammonium chloride | 0.8 |
| 8. Vitamin E acetate | 0.1 |
| 9. Polyethylene glycol 400 | 1.0 |
| 10. Glycerol | 10.0 |
| 11. Aluminum magnesium silicate | 1.2 |
| 12. Antiseptic | 0.5 |
| 13. Perfume | 0.2 |
| 14. Purified water | 33.7 |
| Total | 100.0 |

| | |
|---|---|
| (Note 1) KF-8108: manufactured by Shin-Etsu Chemical Co., Ltd. (Note 2) KF-6028P: manufactured by Shin-Etsu Chemical Co., Ltd. (Note 3) KSP-102: manufactured by Shin-Etsu Chemical Co., Ltd. | |

### <Preparation of cosmetics>

A: The components 1 to 8, the component 12 were uniformly mixed.
B: The components 9 to 11, and 14 were uniformly mixed.
C: Under stirring, B was gradually added to A and emulsified, and the component 13 was added to the emulsion to obtain a hand cream.

The hand cream obtained as described above was not oily and was smooth, was light in the spreadability, and maintained the skin protection effect.

### (Example 27) O/W cream

| Composition | Mass (%) |
|---|---|
| 1. Dimethylpolysiloxane (6cs) | 9.0 |
| 2. Stearyl-modified acrylic silicone resin (Note 1) | 8.0 |
| 3. Organopolysiloxane of Example 1 | 3.0 |
| 4. Glyceryl triisostearate | 10.0 |
| 5. Cetanol | 1.0 |
| 6. Stearic acid | 3.0 |
| 7. Glyceryl monostearate | 1.5 |
| 8. Sorbitan sesquioleate | 0.5 |
| 9. Polyoxyethylene sorbitan monooleate | 1.0 |
| 10. Sodium hydroxide (1% by mass aqueous solution) | 10.0 |
| 11. 1,3-butylene glycol | 5.0 |
| 12. Antiseptic | 0.5 |
| 13. Perfume | 0.2 |
| 14. Purified water | 47.3 |
| Total | 100.0 |

| | |
|---|---|
| (Note 1) KP-561P: manufactured by Shin-Etsu Chemical Co., Ltd. | |

### <Preparation of cosmetics>

A: The components 1 to 9 were heated and mixed.
B: The components 10 to 12, and 14 were mixed and heated.
C: Under stirring, B was gradually added to A and emulsified, and cooled, and then the component 13 was added to the emulsion to obtain an O/W cream.

It was confirmed that the O/W cream obtained as described above was not sticky and not oily, and was smooth, was light in the spreadability, and had the refreshing feeling in use.

### (Example 28) O/W cream

| Composition | Mass (%) |
|---|---|
| 1. Polyglyceryl monooleate | 1.0 |
| 2. Cetyl alcohol | 0.5 |
| 3. Stearic acid | 1.0 |
| 4. Glyceryl monostearate | 1.0 |
| 5. Organopolysiloxane of Example 4 | 2.0 |
| 6. Macadamian nut oil | 9.0 |
| 7. Crosslinked dimethylpolysiloxane (Note 1) | 0.5 |
| 8. (Acrylates / alkyl acrylate (C10-30)) cross polymer (Note 2) | 0.2 |
| 9. Methylcellulose | 0.1 |
| 10. Triethanolamine | 0.2 |
| 11. 1,3-butylene glycol | 7.0 |
| 12. Antiseptic | 0.5 |
| 13. Perfume | 0.2 |
| 14. Purified water | 76.8 |
| Total | 100.0 |

| | |
|---|---|
| (Note 1) KSG-16: manufactured by Shin-Etsu Chemical Co., Ltd. (Note 2) Pemulen TR-1: manufactured by Noveon International, Inc. | |

### <Preparation of cosmetics>

A: The components 1 to 7 were heated and uniformly mixed.
B: The components 8 to 12 and 14 were mixed and heated.
C: Under stirring, B was gradually added to A and emulsified, and cooled, and then the component 13 was added to the emulsion to obtain an O/W cream.

It was confirmed that the O/W cream obtained as described above was not sticky and not oily, and was smooth, was light in the spreadability, and maintained freshness of the skin.

### (Example 29) antiperspirant

| Composition | Mass (%) |
|---|---|
| 1. Crosslinked polyether-modified silicone (Note 1) | 7.0 |
| 2. Organopolysiloxane of Example 1 | 5.0 |
| 3. Decamethylcyclopentasiloxane | 11.0 |
| 4. 1,3-butylene glycol | 6.0 |
| 5. Sodium citrate | 0.2 |
| 6. Glycine salt of aluminum zirconium tetrachloride hydrate | 20.0 |
| 7. Purified water | 50.8 |
| Total | 100.0 |

| | |
|---|---|
| (Note 1) KSG-210: manufactured by Shin-Etsu Chemical Co., Ltd. | |

### <Preparation of cosmetics>

A: The components 1 to 3 were uniformly mixed.
B: The components 4 to 7 were uniformly mixed.
C: Under stirring, B was gradually added to A and emulsified to obtain a antiperspirant.

It was confirmed that the antiperspirant obtained as described above was light in the spreadability, did not whiten the skin, and had good duration of antiperspirant effect.

### (Example 30) cleansing cream

| Composition | Mass (%) |
|---|---|
| 1. Dimethylpolysiloxane (6cs) | 5.0 |
| 2. Methylphenylpolysiloxane (Note 1) | 5.0 |
| 3. Liquid paraffin | 6.0 |
| 4. Jojoba oil | 3.0 |
| 5. Organopolysiloxane of Example 2 | 3.0 |
| 6. Branched polyether-modified silicone (Note 2) | 2.0 |
| 7. Dextrin fatty acid ester | 0.8 |
| 8. Aluminum monostearate | 0.2 |
| 9. Aluminum chloride | 1.0 |
| 10. Glycerol | 10.0 |
| 11. Antiseptic | 0.5 |
| 12. Perfume | 0.2 |
| 13. Purified water | 63.3 |
| Total | 100.0 |

| | |
|---|---|
| (Note 1) KF-56: manufactured by Shin-Etsu Chemical Co., Ltd. (Note 2) KF-6028: manufactured by Shin-Etsu Chemical Co., Ltd. | |

### <Preparation of cosmetics>

A: The components 1 to 8 were heated and mixed.
B: The components 9 to 11, and 13 were heated and mixed.
C: Under stirring, B was gradually added to A and emulsified, and cooled, and the component 12 was added to the emulsion to obtain a cleansing cream.

It was confirmed that the cleansing cream obtained as described above was light in the spreadability, was moist and fresh, gave the refreshing feeling in use.

### (Example 31) W/O rouge

| Composition | Mass (%) |
|---|---|
| 1. Acrylic silicone resin dissolved material (Note 1) | 10.0 |
| 2. Stearyl-modified acrylic silicone resin (Note 2) | 2.0 |
| 3. Branched polyether-modified silicone (Note 3) | 1.5 |
| 4. Decamethylcyclopentasiloxane | 15.0 |
| 5. Glyceryl triisostearate | 5.0 |
| 6. Organopolysiloxane of Example 3 | 2.0 |
| 7. Dimethyl distearyl ammonium hectorite | 1.5 |
| 8. Spherical nylon | 3.0 |
| 9. Talc | 4.0 |
| 10. Rouge pigment (Acrylic silicone-treated) (Note 4) | 20.0 |
| 11. Alcohol | 6.0 |
| 12. Perfume | 0.2 |
| 13. Purified water | 29.8 |
| Total | 100.0 |

| | |
|---|---|
| (Note 1) KP-545: manufactured by Shin-Etsu Chemical Co., Ltd. (Note 2) KP-561P: manufactured by Shin-Etsu Chemical Co., Ltd. (Note 3) KF-6028P: manufactured by Shin-Etsu Chemical Co., Ltd. (Note 4) KP-574: manufactured by Shin-Etsu Chemical Co., Ltd., treatment | |

### <Preparation of cosmetics>

A: The components 1 to 7 were warmed and mixed.
B: The components 8 to 10, and the component 12 were uniformly mixed, and mixed with A.
C: The components 11, 13 were mixed.
D: Under stirring, C was gradually added to B and emulsified to obtain a W/O rouge.

The W/O rouge obtained as described above was not sticky and not oily, was light in the spreadability, excellent in adhesion, and also good in the cosmetic durability.

### (Example 32) W/O liquid foundation

| Composition | Mass (%) |
|---|---|
| 1. Crosslinked polyether-modified silicone (Note 1) | 3.0 |
| 2. Crosslinked dimethylpolysiloxane (Note 2) | 5.0 |
| 3. Branched polyether-modified silicone (Note 3) | 2.0 |
| 4. Decamethylcyclopentasiloxane | 25.0 |
| 5. Cetyl isooctanoate | 5.0 |
| 6. Organopolysiloxane of Example 1 | 5.0 |
| 7. Dimethyl distearyl ammonium hectorite | 1.2 |
| 8. foundation pigment (silicone-treated) (Note 4) | 14.0 |
| 9. Acrylic silicone resin dissolved material (Note 5) | 10.0 |
| 10. 1,3-butylene glycol | 5.0 |
| 11. Xanthan gum | 0.1 |
| 12. Sodium citrate | 0.2 |
| 13. Sodium chloride | 0.5 |
| 14. Antiseptic | 0.5 |
| 15. Perfume | 0.2 |
| 16. Purified water | 23.3 |
| Total | 100.0 |

| | |
|---|---|
| (Note 1) KSG-210: manufactured by Shin-Etsu Chemical Co., Ltd. (Note 2) KSG-15: manufactured by Shin-Etsu Chemical Co., Ltd. (Note 3) KF-6028P: manufactured by Shin-Etsu Chemical Co., Ltd. (Note 4) KF-9909: manufactured by Shin-Etsu Chemical Co., Ltd., treatment (Note 5) KP-575: manufactured by Shin-Etsu Chemical Co., Ltd. | |

### <Preparation of cosmetics>

A: Part of the component 4 and the component 9 were mixed, and the component 8 was dispersed in the mixture.
B: The components 1 to 3 and the remainder of the component 4, and the component 5 to 7 were uniformly mixed.
C: The components 10 to 14 and the component 16 were uniformly mixed.
D: Under stirring, C was gradually added to B and emulsified, and A and the component 15 were added to the emulsion to obtain a W/O liquid foundation.

The W/O liquid foundation obtained as described above was not sticky and not oily, was light in the spreadability, and also good in the cosmetic durability, and also free from the secondary adhesion.

### (Example 33) W/O cream

| Composition | Mass (%) |
|---|---|
| 1. Crosslinked alkyl polyether-modified silicone (Note 1) | 3.0 |
| 2. Crosslinked alkyl-modified dimethylpolysiloxane (Note 2) | 4.0 |
| 3. Alkyl-modified branched polyether-modified silicone (Note 3) | 1.0 |
| 4. Meadowfoam oil | 4.5 |
| 5. Jojoba oil | 3.5 |
| 6. Macadamian nut oil | 7.0 |
| 7. Organopolysiloxane of Example 5 | 4.0 |
| 8. Hybrid silicone composite powder (Note 4) | 3.0 |
| 9. 1,3-butylene glycol | 9.0 |
| 10. Glycine | 3.0 |
| 11. Sodium citrate | 0.2 |
| 12. Sodium chloride | 0.5 |
| 13. Antiseptic | 0.5 |
| 14. Perfume | 0.2 |
| 15. Purified water | 56.6 |
| Total | 100.0 |

| | |
|---|---|
| (Note 1) KSG-340: manufactured by Shin-Etsu Chemical Co., Ltd. (Note 2) KSG-44: manufactured by Shin-Etsu Chemical Co., Ltd. (Note 3) KF-6038: manufactured by Shin-Etsu Chemical Co., Ltd. (Note 4) KSP-100: manufactured by Shin-Etsu Chemical Co., Ltd. | |

### <Preparation of cosmetics>

A: The components 1 to 8 were uniformly mixed.
B: The components 9 to 13, and the component 15 were uniformly mixed.
C: Under stirring, B was gradually added to A and emulsified, and the component 14 was added to the emulsion to obtain a W/O cream.

It was confirmed that the W/O cream obtained as described above was not sticky and not oily, and maintained a moist feeling.

### (Example 34) cuticle coat

| Composition | Mass (%) |
|---|---|
| 1. Polyether-modified silicone (Note 1) | 3.0 |
| 2. Polyether-modified silicone (Note 2) | 2.0 |
| 3. PEG-40 hydrogenated hardened castor oil | 1.0 |
| 4. Organopolysiloxane of Example 1 | 3.0 |
| 5. Silicone gum dissolved material (Note 3) | 40.0 |
| 6. Decamethylcyclopentasiloxane | 40.0 |
| 7. Alcohol | 4.3 |
| 8. Antiseptic | 0.5 |
| 9. Perfume | 0.2 |
| 10. Purified water | 6.0 |
| Total | 100.0 |

| | |
|---|---|
| (Note 1) KF-6011: manufactured by Shin-Etsu Chemical Co., Ltd. (Note 2) KF-6013: manufactured by Shin-Etsu Chemical Co., Ltd. (Note 3) KF-9028: manufactured by Shin-Etsu Chemical Co., Ltd. | |

### <Preparation of cosmetics>

A: The components 1 to 3, and 7 to 10 were uniformly mixed.
B: The components 4 to 6 were uniformly mixed.
C: Under stirring, B was added to A and emulsified to obtain a cuticle coat.

It was confirmed that the cuticle coat obtained as described above was light in the spreadability, suppressed overly dry of the hair, gave the hair gloss and smoothness.

### (Example 35) hair treatment

| Composition | Mass (%) |
|---|---|
| 1. Silicone gum dissolved material (Note 1) | 5.0 |
| 2. Diphenyl dimethicone (Note 2) | 4.0 |
| 3. Organopolysiloxane of Example 4 | 1.0 |
| 4. Cetyl octanoate | 1.0 |
| 5. Cetyl alcohol | 0.5 |
| 6. Polyether-modified silicone (Note 3) | 1.0 |
| 7. PEG-60 hydrogenated hardened castor oil | 1.0 |
| 8. Glyceryl monostearate | 0.5 |
| 9. Carboxyvinyl polymer (1% by mass aqueous solution) | 25.0 |
| 10. Xanthan gum (1% by mass aqueous solution) | 7.0 |
| 11. 1,3-butylene glycol | 5.0 |
| 12. Alcohol | 7.0 |
| 13. Antiseptic | 0.5 |
| 14. Perfume | 0.2 |
| 15. Purified water | 41.3 |
| Total | 100.0 |

| | |
|---|---|
| (Note 1) MK-15H: manufactured by Shin-Etsu Chemical Co., Ltd. (Note 2) KF-54: manufactured by Shin-Etsu Chemical Co., Ltd. (Note 3) KF-6013: manufactured by Shin-Etsu Chemical Co., Ltd. | |

### <Preparation of cosmetics>

A: The components 1 to 8 were heated and dissolved.
B: The components 11 to 15 were heated and dissolved.
C: Under stirring, B was added to A and emulsified, and further the components 9 and 10 were added to the emulsion to obtain a hair treatment.

It was confirmed that the hair treatment obtained as described above was light in the spreadability, gave the hair gloss and smoothness.

It must be stated here that the present invention is not restricted to the embodiments shown by Examples. The embodiments shown by Examples are merely examples so that any embodiments composed of substantially the same technical concept as disclosed in the claims of the present invention and expressing a similar effect are included in the technical scope of the present invention.

## Claims

1. A cosmetic comprising a urea group-containing organopolysiloxane represented by the following average composition formula (1), 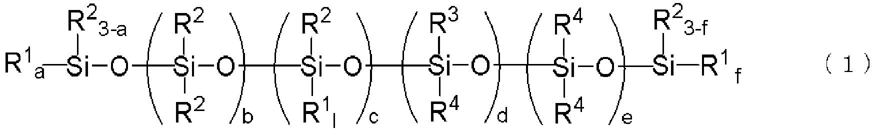
in the formula (1), R¹ is -R⁵-NHCONH₂ (R⁵ is a divalent hydrocarbon group having 2 to 10 carbon atoms), R²s are each independently any of a hydrogen atom, a hydroxy group, an alkoxy group having 1 to 3 carbon atoms, an alkyl group having 1 to 30 carbon atoms, a fluoroalkyl group having 1 to 30 carbon atoms, an aryl group having 6 to 30 carbon atoms, and an aralkyl group having 7 to 30 carbon atoms, R³ is R¹ or R², and R⁴ is an organopolysiloxane segment represented by the following average composition formula (2), 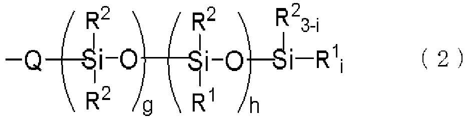
in the formula (2), R¹ and R² are as described above, and Q is an oxygen atom or a divalent hydrocarbon group having 1 to 3 carbon atoms,
in the average composition formula (1) and the general formula (2), "a", "f" and "i" are each independently 0 to 3, "b" is 0 to 5000, "c" is 0 to 500, "d" is 0 to 100, "e" is 0 to 100, "g" is 0 to 5000, and "h" is 0 to 500; provided that, the urea group-containing organopolysiloxane represented by the formula (1) has one or more R¹ in the molecule, when "c" is 0, "a" + "d" + "f" + "h" + "i" is 1 or more.

2. The cosmetic according to claim 1, further comprising water and being in a form of an emulsion system of a water-in-oil emulsion or an oil-in-water emulsion.

3. The cosmetic according to claim 1 or claim 2, further comprising powder.

4. The cosmetic according to any one of claim 1 to claim 3, wherein the cosmetic is a skin care cosmetic, a makeup cosmetic, a hair cosmetic, an antiperspirant cosmetic, or an ultraviolet protection cosmetic.

## Patentansprüche

1. Ein Kosmetikprodukt, das ein Ureagruppen enthaltendes Organopolysiloxan umfasst, das durch die folgende durchschnittliche Konstitutionsformel (1) dargestellt wird, 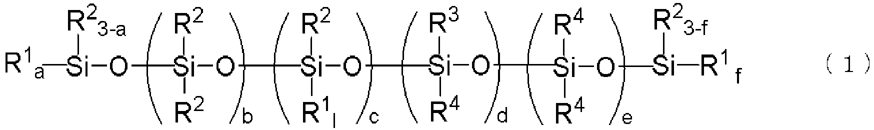
in der Formel (1) ist R¹ -R⁵-NHCONH₂ (R⁵ ist eine divalente Kohlenwasserstoffgruppe mit 2 bis 10 Kohlenstoffatomen), jeder der Reste R² ist unabhängig voneinander ein Wasserstoffatom, eine Hydroxygruppe, eine Alkoxygruppe mit 1 bis 3 Kohlenstoffatomen, eine Alkylgruppe mit 1 bis 30 Kohlenstoffatomen, eine Fluoralkylgruppe mit 1 bis 30 Kohlenstoffatomen, eine Arylgruppe mit 6 bis 30 Kohlenstoffatomen oder eine Aralkylgruppe mit 7 bis 30 Kohlenstoffatomen, R³ ist R¹ oder R² und R⁴ ist ein Organopolysiloxan-Segment, das durch die folgende durchschnittliche Konstitutionsformel (2) dargestellt wird, 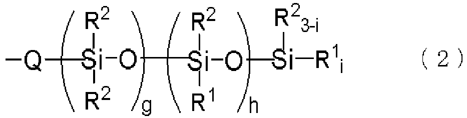
in Formel (2) sind R¹ und R² wie oben beschrieben, und Q ist ein Sauerstoffatom oder eine divalente Kohlenwasserstoffgruppe mit 1 bis 3 Kohlenstoffatomen,
in der durchschnittlichen Konstitutionsformel (1) und der allgemeinen Formel (2) sind "a", "f" und "i" jeweils unabhängig voneinander 0 bis 3, "b" ist 0 bis 5000, "c" ist 0 bis 500, "d" ist 0 bis 100, "e" ist 0 bis 100, "g" ist 0 bis 5000 und "h" ist 0 bis 500; vorausgesetzt, dass das durch die Formel (1) dargestellte eine Harnstoffgruppe enthaltende Organopolysiloxan ein oder mehrere R¹ im Molekül aufweist, dann gilt wenn "c" 0 ist, dann ist "a" + "d" + "f" + "h" + "i" 1 oder mehr.

2. Das Kosmetikprodukt nach Anspruch 1, außerdem Wasser umfassend und in Form eines Emulsionssystems einer Wasser-in-Öl-Emulsion oder einer Öl-in-Wasser-Emulsion vorliegend.

3. Das Kosmetikprodukt nach Anspruch 1 oder Anspruch 2, außerdem Pulver umfassend.

4. Das Kosmetikprodukt nach einem der Ansprüche 1 bis 3, wobei das Kosmetikprodukt ein Hautpflegekosmetikprodukt, ein Make-up-Kosmetikprodukt, ein Haarkosmetikprodukt, ein Anti-transpirant-Kosmetikprodukt oder ein UV-Schutz-Kosmetikprodukt ist.

## Revendications

1. Produit cosmétique comprenant un organopolysiloxane contenant un groupe urée représenté par la formule de composition moyenne (1) suivante 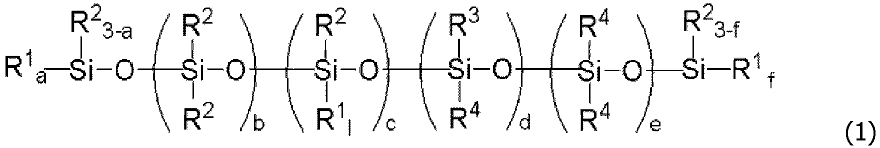
dans la formule (1) R¹ est -R⁵-NHCONH₂ (R⁵ est un groupe hydrocarboné divalent ayant 2 à 10 atomes de carbone), les R² sont chacun indépendamment un atome d'hydrogène, un groupe hydroxy, un groupe alcoxy ayant 1 à 3 atomes de carbone, un groupe alkyle ayant 1 à 30 atomes de carbone, un groupe fluoroalkyle ayant 1 à 30 atomes de carbone, un groupe aryle ayant 6 à 30 atomes de carbone et un groupe aralkyle ayant 7 à 30 atomes de carbone, R³ est R¹ ou R², et R⁴ est un segment organopolysiloxane représenté par la formule de composition moyenne (2) suivante 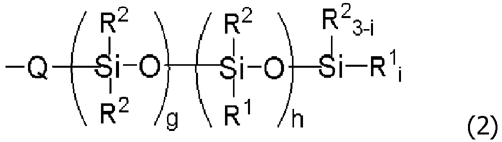
dans la formule (2) R¹ et R² sont tels que décrits ci-dessus, et Q est un atome d'oxygène ou un groupe hydrocarboné divalent ayant 1 à 3 atomes de carbone,
dans la formule de composition moyenne (1) et la formule générale (2), « a », « f » et « i » sont chacun indépendamment 0 à 3, « b » est 0 à 5 000, « c » est 0 à 500, « d » est 0 à 100, « e » est 0 à 100, « g » est 0 à 5 000, et « h » est 0 à 500 ; à condition que l'organopolysiloxane contenant un groupe urée représenté par la formule (1) ait un ou plusieurs R¹ dans la molécule, lorsque « c » est 0, « a » + « d » + « f » + « h » + « i » est 1 ou plus.

2. Produit cosmétique selon la revendication 1, comprenant en outre de l'eau et se présentant sous la forme d'un système d'émulsion d'une émulsion eau dans huile ou d'une émulsion huile dans eau.

3. Produit cosmétique selon la revendication 1 ou la revendication 2, comprenant en outre de la poudre.

4. Produit cosmétique selon l'une quelconque des revendications 1 à 3, dans lequel le produit cosmétique est un produit cosmétique de soin de la peau, un produit cosmétique de maquillage, un produit cosmétique capillaire, un produit cosmétique anti-transpirant ou un produit cosmétique de protection contre les ultraviolets.
